(19) Europäisches Patentamt
European Patent Office
Office européen des brevets

(11) Numéro de publication: **0 429 341 A2**

# (12) DEMANDE DE BREVET EUROPEEN

(21) Numéro de dépôt: **90403219.0**

(22) Date de dépôt: **14.11.90**

(51) Int. Cl.[5]: **C07D 275/06**, A61K 31/425, C07D 513/06, C07D 209/90, C07D 471/06, C07D 235/26, C07D 417/14, C07D 417/12, C07D 401/14, C07D 417/06, C07D 401/06

(30) Priorité: **20.11.89 FR 8915177**
**26.12.89 FR 8917164**
**21.06.90 FR 9007774**

(43) Date de publication de la demande:
**29.05.91 Bulletin 91/22**

(84) Etats contractants désignés:
**AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Gueremy, Claude**
**3 rue Daumesnil**
**F-78800 Houilles(FR)**
Inventeur: **Malleron, Jean-Luc**
**2 allée Renoir**
**F-91460 Marcoussis(FR)**
Inventeur: **Mignani, Serge**
**14 allée Jean-Baptiste Clément**
**F-93190 Livry-Gargan(FR)**

(74) Mandataire: **Savina, Jacques et al**
**RHONE-POULENC SANTE, Service Brevets,**
**20 Avenue Raymond Aron**
**F-92165 Antony Cédex(FR)**

## (54) Dérivés hétérocycliques, leur préparation et les médicaments les contenant.

(57) Composés de formule :
$R_1 - (CH_2)_n - Het$
dans laquelle
- $R_1$ représente un reste de formule

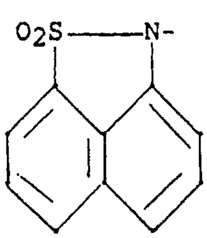

(A)

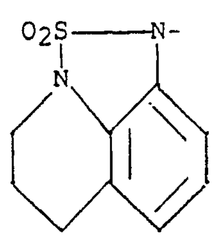

(B)

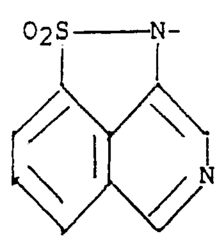

(C)

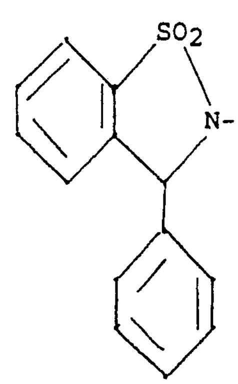

(D)

EP 0 429 341 A2

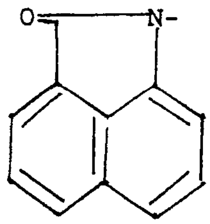

(E)

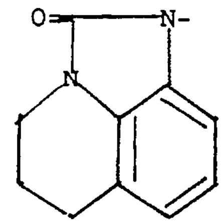

(F)

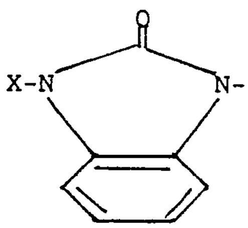

(G)

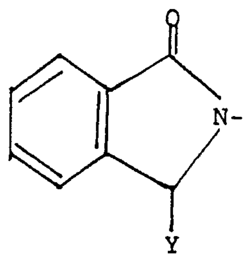

(H)

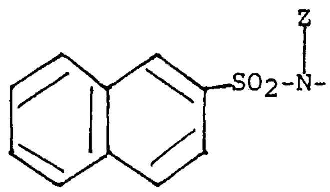

(J)

- X représente un atome d'hydrogène ou un radical alkyle,
- Y représente un atome d'hydrogène ou un radical alkyle ou alcoxy,
- Z représente un radical alkyle,
- n est égal à 2 ou 3
- Het représente un radical pipéridino substitué (en position-4 par un radical indénylidène-1, indényl-1, indanyl-1 ou par une chaîne $-(CH_2)_m-R_2-$ ou $=CH-R_2$), tétrahydro-1,2,3,6 pyridyl-1 substitué (en position -4 par une chaîne $-(CH_2)_m-R_2$) ou pipérazinyl-1 substitué (en position -4 par une chaîne $-(CH_2)_m-R_2$),
- m est égal à 1 ou 2,
- $R_2$ représente un radical indolyl-2 ou -3 éventuellement substitué, indanyl-1 ou -2, indényl-1 ou -2, quinolyl-3, chromanyl-3, tétrahydro-1,2,3,4 5H pyrido [4,3-b] indolyl-2 éventuellement substitué, (tétrahydro-1,2,3,4 quinolyl)-3, (tétrahydro-1,2,3,4 naphtyl)-2 éventuellement substitué, indolyl-1, leur préparation et les médicaments les contenant.

## DERIVES HETEROCYCLIOUES, LEUR PREPARATION ET LES MEDICAMENTS LES CONTENANT

La présente invention concerne des composés de formule:

$R_1 - (CH_2)_n - Het$ (I)

leur préparation et les médicaments les contenant.

Dans la formule (I),

- $R_1$ représente un reste de formule :

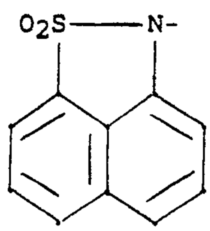

(A)

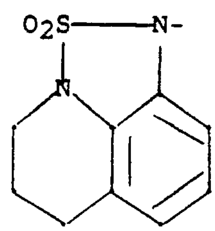

(B)

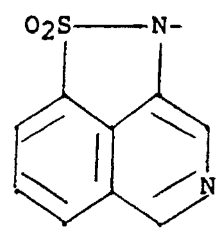

(C)

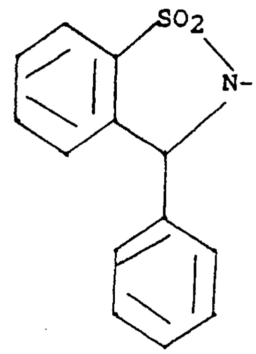

(D)

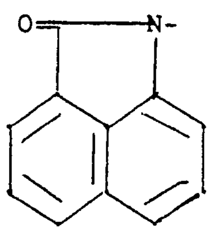

(E)

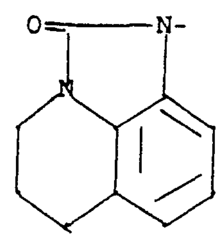

(F)

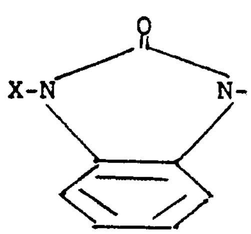

(G)

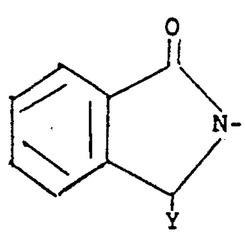

(H)

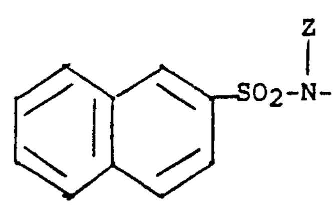

(J)

- X représente un atome d'hydrogène ou un radical alkyle,
- Y représente un atome d'hydrogène ou un radical alkyle ou alcoxy,
- Z représente un radical alkyle,
- n est égal à 2 ou 3,
- Het représente un radical pipéridino substitué (en position -4 par un radical indénylidène-1, indényl-1, indanyl-1 ou par une chaîne $-(CH_2)_m-R_2$ ou $=CH-R_2$), tétrahydro-1,2,3,6 pyridyl-1 substitué (en position -4 par une chaîne $-(CH_2)_m-R_2$) ou pipérazinyl-1 substitué (en position -4 par une chaîne $-(CH_2)_m-R_2$),
- m est égal à 1 ou 2,
- $R_2$ représente un radical indolyl-2 ou -3 (éventuellement substitué par un atome d'halogène et/ou sur

l'atome d'azote par un radical alkyle), indanyl-1 ou -2, indényl-1 ou -2, quinolyl-3, chromanyl-3, tétrahydro-1,2,3,4 5H pyrido [4,3-b] indolyl-2 (éventuellement substitué par un atome d'halogène), (tétrahydro-1,2,3,4 quinolyl)-3, (tétrahydro-1,2,3,4, naphtyl)-2 (éventuellement substitué par un atome d'halogène), indolyl-1.

Dans les définitions qui précèdent et celles qui seront citées ci-après, les radicaux alkyle et alcoxy contiennent 1 à 4 atomes de carbone en chaîne droite ou ramifiée.

L'invention concerne également les sels des composés de formule (I) avec les acides minéraux ou organiques.

Dans la formule (I) les atomes d'halogène sont de préférence des atomes de fluor.

Les composés de formule (I) contenant au moins un centre asymétrique présentent des formes isomères. Les racémiques et les énantiomères de ces composés font également partie de l'invention.

Les composés de formule (I) à l'exception de ceux pour lesquels ri représente un reste de formule (G) dans laquelle X représente un atome d'hydrogène peuvent être préparés par action d'un dérivé halogéné de formule :

$R_1 - (CH_2)_n - Hal$ (II)

dans laquelle $R_1$ a les mêmes significations que précédemment, n a les mêmes significations que dans la formule (I) et Hal représente un atome d'halogène (chlore, brome ou iode de préférence), sur un dérivé de formule :

H - Het (III)

dans laquelle Het a les mêmes significations que dans la formule (I).

Cette réaction s'effectue généralement au sein d'un solvant inerte tel que le tétrahydrofuranne, le diméthylformamide ou un mélange de ces solvants, en présence d'une base telle qu'un bicarbonate de métal alcalin ou une amine tertiaire telle qu'une trialkylamine, à la température d'ébullition du solvant.

Les dérivés halogénés de formule (II) peuvent être obtenus par action d'un dérivé de formule :

$R_1 - H$ (IV)

dans laquelle $R_1$ a les mêmes significations que précédemment, sur un dérivé dihalogéné de formule :

$Hal - (CH_2)_n - X$ (V)

dans laquelle Hal et X représentent un atome d'halogène (chore, brome ou iode de préférence) et n a les mêmes significations que dans la formule (I).

Cette réaction s'effectue généralement au sein d'un solvant inerte tel que le diméthylformamide en présence d'hydrure de sodium, à une température comprise entre 20°C et la température d'ébullition du solvant.

Les dérivés de formule (IV) sont commercialisés ou peuvent être obtenus par application ou adaptation des méthodes décrites dans les exemples.

Les dérivés de formule (III) peuvent être obtenus par application ou adaptation des méthodes décrites par C. GUEREMY et coll., J. Med. Chem., 23, 1306 (1980) ; C.S. RUNTY et coll., gazz. Chem. Ital., 81, 613 (1951) ; A.O.M. STOPPANI, Revista Argentina Microbiologica, 19, 121 (1987) ; C. GUEREMY et coll. brevet EP 42322, A.P. GRAY, J. Am. Chem. Soc., 79, 3554 (1957) ; R.T. BORCHARDT et Coll., J. Het. Chem., 24, 1499 (1987) ; V. SNIECKUS, Can. J. Chem., 51, 792 (1973) et dans les exemples.

Les composés de formule (I) pour lesquels $R_1$ représente un reste de formule (G) dans laquelle X représente un atome d'hydrogène peuvent être préparés par hydrolyse d'un dérivé de formule :

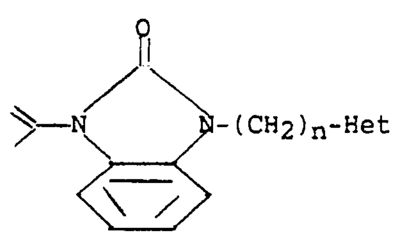

(VI)

dans laquelle n et Het ont les mêmes significations que dans la formule (I).

Cette hydrolyse s'effectue en milieu acide (acide sulfurique, acide chlorhydrique par exemple) au sein d'un solvant inerte tel qu'un alcool (méthanol, éthanol par exemple) à une température comprise entre 20°C et la température d'ébullition du solvant. Les composés de formule (VI) peuvent être obtenus par application ou adaptation de la méthode décrite dans les exemples. Les composés de formule (I) pour lesquels $R_2$ représente un radical indolyl-2 ou -3 substitué sur l'atome d'azote par un radical alkyle et éventuellement substitué par un atome d'halogène peuvent également être obtenus par alkylation d'un

composé de formule (I) correspondant pour lequel $R_2$ représente un radical indolyl-2 ou 3 éventuellement substitué par un atome d'halogène.

Cette alkylation s'effectue généralement au moyen d'une halogènure d'alkyle en présence d'une base telle qu'un hydrure de métal alcalin, au sein d'un solvant inerte tel que le diméthylformamide, le tétrahydrofuranne ou le dioxanne, à une température voisine de 20° C.

Les composés de formule (I) pour lesquels $R_1$ représente un reste de formule (H) dans laquelle Y représente un atome d'hydrogène peuvent également être obtenus par réduction d'un dérivé de formule :

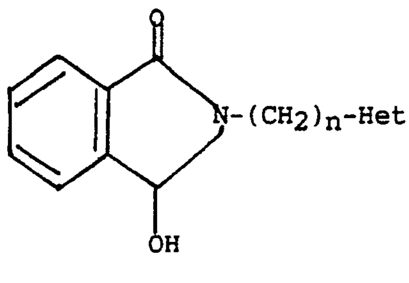

(VII)

dans laquelle n et Het ont les mêmes significations que dans la formule (I).

Cette réduction s'effectue de préférence, au moyen d'un agent réducteur tel que le zinc, en présence d'un acide tel que l'acide acétique, à une température voisine de 120° C.

Les composés de formule (I) pour lesquels Het représente un radical pipéridino substitué en position-4 par un radical indénylidène-1 peuvent également être préparés par réaction d'un dérivé de formule :

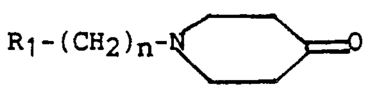

(VIII)

dans laquelle $R_1$ et n ont les mêmes significations que dans la formule (I) avec un dérivé métallique de l'indène.

Cette réaction s'effectue au sein d'un solvant inerte tel que le tétrahydrofuranne ou l'oxyde de diéthyle par exemple à une température comprise entre -78° et 30° C. Code dérivé métallique, on utilise de préférence le dérivé lithié.

Les dérivés de formule (VIII) peuvent être obtenus par application ou adaptation de la méthode décrite dans les exemples.

Les énantiomères des composés de formule (I) contenant au moins un site asymétrique peuvent être obtenus par synthèse à partir des précurseurs chiraux ou par dédoublement des racémiques par exemple par chromatographie sur colonne chirale selon W.H. PIRCKLE et coll., asymetric synthesis, vol 1, Academic Press (1983).

Les mélanges réactionnels obtenus par les divers procédés précédemment décrits sont traités selon des méthodes classiques physiques (évaporation, extraction, distillation, cristallisation, chromatographie ...) ou chimiques (formation des sels ...).

Les composés de formule (I), sous forme de base libre peuvent être transformés en sel d'addition avec les acides par action d'un acide minéral ou organique dans un solvant organique tel qu'un alcool, une cétone, un solvant chloré ou un éther.

Les composés de formule (I) présentent des propriétés pharmacologiques intéressantes. Ces composés sont des inhibiteurs de la recapture de 5HT et sont donc utiles dans le traitement de la dépression, des troubles obsessionnels, de l'obésité, des troubles de la conduite alimentaire notamment les excès d'alcool, ainsi que des troubles de l'apprentissage et de la mémoire, des attaques de panique et de la douleur.

L'affinité de ces produits vis-à-vis du site de liaison de la paroxétine (reflet de l'inhibition de la recapture de 5HT) a été mesurée par la méthode de E. HABERT et coll., Eur. J. Pharmacol., 118, 107 (1985).

Dans ce test, les composés de formule (I) présentent une $CI_{50}$ inférieure à 25nM.

Les composés de formule (I) présentent une toxicité faible. Ils sont généralement atoxiques à 300 mg/kg par voie orale chez la souris en administration unique.

Sont particulièrement intéressants les composés de formule (I) pour lesquels Het représente un radical

pipéridino substitué en position-4 par une chaîne $-(CH_2)_m-R_2$ et $R_2$ représente un radical (fluoro-5 indolyl)-3.

D'un intérêt particulier sont les produits suivants :

- {[((fluoro-5 indolyl-3) méthyl)-4 pipéridino]-2 éthyl}-1 benzo[cd] indolone-2
- {[((fluoro-5 indolyl-3) méthyl)-4 pipéridino]-3 propyl}-2 naphto [1,8-cd] isothiazole dioxyde-1,1
- {[((fluoro-5 indolyl-3) méthyl)-4 pipéridino]-2 éthyl}-2 2H-isothiazolo[3,4,5-de] isoquinoléine dioxyde-1,1
- {[((fluoro-5 indolyl-3) méthyl)-4 pipéridino]-2 éthyl}-1 dihy dro-5,6 1H,4H-1,2,5-thiadiazolo [4,3,2-ij] quinoléine dioxyde-2,2
- {[((fluoro-5 indolyl-3) méthyl)-4 pipéridino]-2 éthyl}-2 naphto [1,8-cd] isothiazole dioxyde-1,1
- {[(fluoro-5 indolyl-3) méthyl)-4 pipéridino]-2 éthyl}-1 oxo-2 tétrahydro-1,2,5,6 4H-imidazo [4,5,1-ij] quinoléine
- méthyl-3 {[((fluoro-5 indolyl-3) méthyl)-4 pipéridino]-2 éthyl}-1 2H-benzimidazolinone-2
- {[((fluoro-5 indolyl-3) méthyl)-4 pipéridino]-2 éthyl)-2 méthoxy-3 isoindolinone-(RS)
- {[((fluoro-5 indolyl-3) méthyl)-4 pipéridino]-2 éthyl}-2 isoindolinone-(RS)

Pour l'emploi thérapeutique, il peut être fait usage des composés de formule (I) tels quels ou à l'état de sels pharmaceutiquement acceptables.

Comme sels pharmaceutiquement acceptables, peuvent être notamment cités les sels d'addition avec les acides minéraux tels que chlorhydrates, sulfates, nitrates, phosphates ou organiques tels que acétates, propionates, succinates, oxalates, benzoates, fumarates, maléates, méthanesulfonates, iséthionates, théophillineacétate, salicylates, phénolphtalinates, méthylène-bis-8-oxynaphtoates ou des dérivés de substitution de ces dérivés.

Les exemples suivants donnés à titre non limitatif montrent comment l'invention peut être mise en pratique.

## EXEMPLE 1

Un mélange de 5,4 g de (chloro-2 éthyl)-2 naphto [1,8-cd] isothiazole dioxyde-1,1, 4,6 g d'[(indolyl-3)-2 éthyl]-4 pipéridine et 1,7 g de bicarbonate de sodium dans 1 20cm3 de tétrahydrofuranne et 120 cm3 de diméthylformamide est chauffé 8 heures à ébullition puis refroidi à une température voisine de 20° C. Le précipité formé est séparé par filtration, le filtrat est évaporé à sec à 40° C sous pression réduite (20 mm de mercure ; 2,7 kPa). Le résidu obtenu est purifié par flash-chromatographie sur colonne de silice, sous courant d'azote à moyenne pression (0,5-1,5 bar) avec de l'acétate d'éthyle comme éluant. Après recristallisation dans l'acétonitrile bouillant, on obtient 2,6 g d'{[((indolyl-3)-2 éthyl)-4 pipéridino]-2 éthyl-2} naphto [1,8-cd] isothiazole dioxyde-1,1 fondant à 169° C.

Le (chloro-2 éthyl)-2 naphto [1,8-cdj isothiazole dioxyde-1,1 peut être préparé de la manière suivante : à 19,2 g d'hydrure de sodium en dispersion à 50% dans de l'huile de vaseline, traversé par un courant d'argon, on ajoute en 2 heures une solution de 82 g de naphtosultam- 1,8 dans 1000 cm3 de diméthylformamide, en maintenant à une température voisine de 35° C . Le milieu réactionnel est agité 1 heure à une température voisine de 20° C puis on ajoute 35,2 cm3 de bromo-1 chloro-2 éthane. L'agitation est maintenue durant 15 heures à une température voisine de 20° C. Le mélange réactionnel est concentré à sec à 70° C sous pression réduite (0,5 - le mercure ; 0,07kPa) et le résidu est repris par 1000 cm3 de dichlorométhane es filtré . Le filtrat est lavé par 4 fois 1000 cm3 d'eau et la phase organique est décantée, séchée sur du sulfate de magnésium anhydre et évaporée à sec à 40° C sous pression réduite (20 mm de mercure ; 2,7 kPa). L'huile obtenue est purifiée par flash-chromatographie sur colonne de silice , sous courant d'argon à moyenne pression (0,1-1,5 bar) avec du dichlorométhane comme éluant. On obtient 70,2g de (chloro-2 éthyl)-2 naphto [1,8-cd] isothiazole dioxyde-1,1 fondant à 96° C.

L'[(indolyl-3)-2 éthyl]-4 pipéridine peut être préparée selon la méthode décrite par C.GUEREMY et coll., J. Med. Chem., 23, 1306 (1980).

## EXEMPLE 2

A 0,27 g d'hydrure de sodium en dispersion à 50% dans l'huile de vaseline, sous courant d'argon, on ajoute, en 15 minutes, un mélange de 2,3 g de {[((fluoro-5 indolyl-3) méthyl)-4 pipéridino]-2 éthyl}-2 naphto[1,8-cd] isothiazole dioxyde-1,1 dans 40 cm3 de diméthylformamide, en maintenant la température voisine de 20° C. Le milieu réactionnel est agité 1 heure à une température proche de 20° C puis on ajoute en 15 minutes, une solution de 0,4 cm3 d'iodure de méthyle dans 20 cm3 de dioxanne. L'agitation est maintenue durant heures à une température proche de 20° C. Le mélange réactionnel est repris par 25 cm3 d'eau distillée et la phase organique est extraite par 4 fois 50 cm3 de dichlorométhane puis lavée par 25 cm3 d'eau distillée, séchée sur du sulfate de magnésium anhydre, filtrée et concentrée à sec à 40° C sous

pression réduite (20 mm de mercure ; 2,7 kPa). Le résidu obtenu est purifié par flash-chromatographie sur colonne de silice, sous courant d'argon, à moyenne pression (0,5-1,5 bar) avec du dichlorométhane puis un mélange d'acétate d'éthyle et de méthanol (97-3 en volume) comme éluant. On obtient 0,8 g de {[((fluoro-5 méthyl-1 indolyl-3) méthyl)-4 pipéridino]-2 éthyl}-2 naphto[1,8-cd] isothiazole dioxyde-1,1 sous forme d'une huile jaune que l'on transforme en oxalate acide fondant à 150-152° C.

Le {[((fluoro-5 indolyl-3) méthyl)-4 pipéridino]-2 éthyl}-2 naphto[1,8-cd] isothiazole dioxyde-1,1 peut être préparé de la manière suivante : un mélange de 5,3 g de (chloro-2 éthyl)-2 naphto [1,8-cd] isothiazole dioxyde-1,1, 4,6 g de [(fluoro-5 indolyl-3) méthyl]-4 pipéridine et 1,68 g de bicarbonate de sodium dans 120 cm3 de tétrahydrofuranne et 120 cm3 de diméthylformamide est chauffé 48 heures à ébullition puis refroidi à une température voisine de 20° C. Le précipité formé est séparé par filtration et le filtrat est évaporé à sec à 40° C sous pression réduite (20 mm de mercure ; 2,7 kPa). Le résidu obtenu est purifié par flash-chromatographie sur colonne de silice, sous courant d'azote à moyenne pression (0,5-1,5 bar) avec de l'acétate d'éthyle comme éluant. On obtient 4,4 g d'une huile brune. 2 g de cette huile sont recristallisés dans 15 cm3 d'acétonitrile bouillant. On obtient ainsi 1,6 g de {[(fluoro-5 indolyl-3) méthyl)-4 pipéridino]-2 éthyl}-2 naphto [1,8-cd] isothiazole dioxyde-1,1 fondant à 161° C.

La [(fluoro-5 indolyl-3) méthyl]-4 pipéridine peut être préparée selon la méthode décrite par C.GUEREMY et coll., J. Med. Chem., 23, 1306 (1980).

EXEMPLE 3

On opère comme dans l'exemple 1 à partir de 2,7 g de (chloro-2 éthyl)-2 naphto[1,8-cd] isothiazole dioxyde-1,1 , de 2,5 g de [(fluoro-5 indolyl-3)-2 éthyl]-4 pipéridine et de 0,8 g de bicarbonate de sodium dans un mélange de 80 cm3 de diméthylformamide et de 40 cm3 de tétrahydrofuranne. Le mélange est chauffé 9 heures à ébullition puis refroidi à une température voisine de 20° C . Après purification par flash-chromathographie sur colonne de silice, sous courant d'azote, à moyenne pression (0,5-1,5 bar) avec de l'acétate d'éthyle comme éluant et recristallisation dans 15 cm3 d'acétonitrile bouillant , on obtient 1,7 g de {[((fluoro-5 indolyl-3)-2 éthyl)-4 pipéridino]-2 éthyl}-2 naphto[1,8-cd] isothiazole dioxyde-1,1 fondant à 164° C.

La [(fluoro-5 indolyl-3)-2 éthyl]-4 pipéridine peut être préparée selon la méthode décrite par C.GUEREMY et coll., J. Med. Chem., 23, 1306 (1980).

EXEMPLE 4

On opère comme dans l'exemple 1 à partir de 2 g de (chloro-2 éthyl)-2 naphto[1,8-cd] isothiazole dioxyde-1,1, de 2 g de chlorhydrate de [(fluoro-5 indolyl-3) méthyl]-4 tétrahydro-1,2,3,6 pyridine et de 1,3 g de bicarbonate de sodium dans un mélange de 70 cm3 de diméthylformamide et de 45 cm3 de tétrahydrofuranne . Le mélange est chauffé 20 heures à ébullition puis refroidi à une température voisine de 20° C . Après purification par flash-chromathographie sur colonne de silice, sous courant d'azote, à moyenne pression (0,5-1,5 bar) avec du dichlorométhane puis de l'acétate d'éthyle comme éluants, on obtient 0,4 g de {[((fluoro-5 indolyl-3) méthyl)-4 tétrahydro-1,2,3,6 pyridyl-1]-2 éthyl)-2 naphto[1,8-cd] isothiazole dioxyde-1,1 sous forme d'une huile jaune que l'on transforme en oxalate acide fondant à 128° C.

La [(fluoro-5 indolyl-3) méthyl]-4 tétrahydro-1,2,3,6 pyridine peut être préparée de la manière suivante : 11 g de [(fluo ro-5 indolyl-3) méthyl]-4 vinyloxycarbonyl-1 tétrahydro-1,2,3,6 pyridine et 100 cm3 d'une solution de dioxanne chlorhydique 6,3N sont agités 1 heure à une température proche de 20° C. Le mélange est ensuite concentré à sec à 40° C sous pression réduite (20 mm de mercure ; 2,7kPa). L'huile résiduelle est reprise par 40 cm3 d'éthanol et la solution obtenue est chauffée 90 minutes à une température proche de 50° C puis concentrée à sec à 40° C sous pression réduite (20 mm de mercure ; 2,7 kPa). Le résidu est repris par 50 cm3 d'oxyde d'isopropyle ; le précipité formé est séparé par filtration et purifié par flash-chromatographie sur colonne de silice , sous courant d'azote , à moyenne pression (0,5-1,5 bar) avec un mélange de dichlorométhane et de méthanol (90-10 puis 80-20 en volumes) comme éluant . On obtient 3,3 g de [(fluoro-5 indolyl-3) méthyl]-4 tétrahydro-1,2,3,6 pyridine, sous forme d'une huile brune, utilisée telle quelle dans les synthèses ultérieures.

La [(fluoro-5 indolyl-3) méthyl]-4 vinyloxycarbonyl-1 tétrahydro-1,2,3,6 pyridine peut être préparée de la manière suivante : 3,8 cm3 de chloroformiate de vinyle sont ajoutés goutte à goutte à une solution de 9 g de benzyl-1 [(fluoro-5 indolyl-3 ) méthyl]-4 tétrahydro-1,2,3,6 pyridine dans 150 cm3 de dichlorométhane en maintenant la température voisine de 10° C . Le mélange est chauffé 8 heures à ébullition, refroidi à une température proche de 20° C puis concentré à sec à 20° C sous pression réduite (20 mm de mercure ; 2,7 kPa). On obtient 11 g de [(fluoro-5 indolyl-3) méthyl]-4 vinyloxycarbonyl-1 tétrahydro-1,2,3,6 pyridine

utilisée à l'état brut dans les synthèses ultérieures.

La benzyl-1 [(fluoro-5 indolyl-3) méthyl]-4 tétrahydro-1,2,3,6 pyridine peut être préparée de la manière suivante : 1,4 g de borohydrure de sodium sont ajoutés, sous courant d'argon , à une solution de 12 g de bromure de benzyl-1 [(fluoro-5 indolyl-3) méthyl]-4 pyridinium dans 200 cm3 d'éthanol, en gardant la température proche de 25° C. L'agitation est maintenue 36 heures à cette température puis 100 cm3 d'eau distillée sont ajoutés au mélange. La phase organique est extaite par 4 fois 50 cm3 de dichlorométhane, lavée par 50 cm3 d'eau distillée, séchée sur du sulfate de magnésium anhydre et concentrée à sec, à 20° C, sous pression réduite (20 mm de mercure ; 2,7kPa). On obtient 9 g de benzyl-1 [(fluoro-5 indolyl-3) méthyl]-4 tétrahydro-1,2,3,6 pyridine sous forme d'une huile jaune, utilisée à l'état brut dans les synthèses ultérieures.

Le bromure de benzyl-1 [(fluoro-5 indolyl-3) méthyl]-4 tétrahydro-1,2,3,6 pyridine peut être préparé selon la méthode décrite par E.FRIEDERICHS et coll, Arch. Pharm., 308, 209 (1975).

EXEMPLE 5

On opère comme dans l'exemple 1 à partir de 2,4 g de (chloro-2 éthyl)-2 naphto[1,8-cd] isothiazole dioxyde-1,1, de 2,2 g de [(fluoro-5 indolyl-3) méthyl]-4 pipérazine et de 0,8 g de bicarbonate de sodium dans un mélange de 80 cm3 de diméthylformamide et de 40 cm3 de tétrahydrofuranne. Le mélange est chauffé 48 heures à ébullition puis refroidi à une température voisine de 20° C . Après purification par flash-chromathographie sur colonne de silice, sous courant d'azote, à moyenne pression (0,5-1,5 bar) avec un mélange d'acétate d'éthyle et de méthanol (90-10 en volumes) comme éluant et recristallisation dans 7 cm3 d'acétonitrile bouillant, on obtient 1 g de {[((fluoro-5 indolyl-3) méthyl)-4 pipérazinyl-1]-2 éthyl}-2 naphto-[1,8-cd] isothiazole dioxyde-1,1 fondant à 85° C.

La [(fluoro-5 indolyl-3) méthyl]-4 pipérazine peut être préparée selon la méthode décrite par C.S. RUNTY et coll., Gazz. Chem. Ital., 81, 613 (1951) et A.O.M.STOPPANI, Revista Argentina Microbiologia, 19, 121 (1987).

EXEMPLE 6

On opère comme dans l'exemple 1 à partir de 2,8 g de (chloro-3 propyl)-2 naphto[1,8-cdl isothiazole dioxyde-1,1, de 2,3 g de [(fluoro-5 indolyl-3) méthyl]-4 pipéridine et de 0,8 g de bicarbonate de sodium dans un mélange de 90 cm3 de diméthylformamide et de 60 cm3 de tétrahydrofuranne. Le mélange est chauffé 24 heures à ébullition puis refroidi à une température voisine de 20° C. Après purification par flash-chromathographie sur colonne de silice, sous courant d'azote, à moyenne pression (0,5-1,5 bar) avec de l'acétate d'éthyle comme éluant et recristallisation une première fois dans 15 cm3 d'acétonitrile puis une seconde fois dans 5 cm3 d'acétonitrile bouillant, on obtient 2,1 g de {[(( fluoro-5 indolyl-3) méthyl)-4 pipéridino]-3 propyl}-2 naphto[1,8-cd] isothiazole dioxyde-1,1 fondant à 100° C.

Le (chloro-3 propyl)-2 naphto[1,8-cd] isothiazole dioxyde-1,1 peut être préparé de la manière suivante : à 40,2 g d'hydrure de sodium en dispersion à 50 % dans de l'huile de vaseline, sous courant d'argon, on coule en 3 heures et 30 minutes, une solution de 175 g de naphtosultam-1,8 dans 2100 cm3 de diméthylformamide, en maintenant la température comprise entre 20° C et 30° C. Le milieu réactionnel est agité une heure à une température voisine de 20° C puis on ajoute, en 10 minutes, 83 cm3 de bromo-1 chloro-3 propane. L'agitation est maintenue durant 15heures à une température voisine de 20° C. Le mélange réactionnel est concentré à sec à 50° C sous pression réduite (0,5 mm de mercure ; 0,07 kPa). Le résidu est purifié par flash-chromatographie sur colonne de silice, sous courant d'azote, à moyenne pression (0,5-1,5 bar) avec un mélange de dichlorométhane et de cyclohexane (60-40 en volumes) comme éluant. On obtient 165,7 g de (chloro-3 propyl)-2 naphto[1,8-cd] isothiazole dioxyde-1,1 fondant à 78° C.

EXEMPLE 7

On opère comme dans l'exemple 1, à partir de 2,13 g d'(indényl-1 méthyl)-4 pipéridine, 2,94 g de (chloro-2 éthyl)-2 naphto[1,8-cd]isothiazole dioxyde-1,1 et de 0,92 g d'hydrogénocarbonate de sodium dans 50 cm3 de diméthylformamide et 50 cm3 de tétrahydrofuranne. Le mélange est chauffé 12 heures à ébullition puis refroidi à une température voisine de 20° C. Après évaporation à sec à 80° C sous pression réduite (20 mm de mercure; 2,7 KPa), le résidu est repris par 50 cm3 d'eau et extrait deux fois par 75 cm3 de dichlorométhane. Les phases organiques réunies sont séchées sur sulfate de magnésium et amenées à sec à 80° C sous pression réduite (20 mm de mercure ; 2,7 PKa). Le résidu est purifié par flash-chromathographie sur colonne de silice, sous pression d'azote, à moyenne pression (0,5-1,5 bar) avec un

mélange dichlorométhaneméthanol (99-1 en volumes) comme éluant. On obtient 2,7 g d'une huile jaune. On additionne à cette huile 0,54 g d'acide oxalique dans 20 cm3 d'acétone. On isole ainsi 2,5 g d'{[(indényl-1 méthyl)-4 pipéridino]-2 éthyl}-2 naphto[1,8-cd] isothiazole dioxyde-1,1 sous forme d'oxalate fondant à 166 ° C.

L'(indènyl- 1 méthyl) -4 pipéridine peut être préparée selon la méthode décrite par C.GUEREMY et coll., brevet EP 42322.

EXEMPLE 8

On opère comme dans l'exemple 1, à partir de 2,13 g d'(indènyl-1 méthyl)-4 pipéridine, 3,37 g de (chloro-3 propyl)-2 naphto[1,8-cd] isothiazole dioxyde-1,1 et de 1 g d'hydrogénocarbonate de sodium dans 50 cm3 de diméthylformamide et 50 cm3 de tétrahydrofuranne. Le mélange est chauffé 12 heures à ébullition puis refroidi à une température voisine de 20 ° C. Après évaporation à sec à 80 ° C sous pression réduite (20 mm de mercure; 2,7 KPa), le résidu est repris par 50 cm3 d'eau et extrait deux fois par 75 cm3 de dichlorométhane. Les phases organiques réuni es sont séchées sur sulfate de magnésium et amenées à sec à 80 ° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane-méthanol (99-1 en volumes) comme éluant. On obtient 3,3 g d'une huile jaune. On additionne à cette huile 0,67 g d'acide oxalique dans 50 cm3 d'acétone. On isole ainsi 3,5 g d'{[-(indényl-1 méthyl)-4 pipéridino]-3 propyl}-2 naphto[1,8-cd] isothiazole dioxyde-1,1 sous forme d'oxalate fondant à 140 ° C.

EXEMPLE 9

On opère comme dans l'exemple 1, à partir de 2,7 g d'(indanyl-1 méthyl)-4 pipéridine, de 3,2 g de (chloro-2 éthyl)-2 naphto[1,8-cd]isothiazole dioxyde-1,1 et de 2 g d'hydrogénocarbonate de sodium dans 50 cm3 de diméthylformamide et 50 cm3 de tétrahydrofuranne. Le mélange est chauffé 12 heures à ébullition puis refroidi à une température voisine de 20 ° C. Après évaporation à sec à 80 ° C sous pression réduite (20 mm de mercure; 2,7 KPa), le résidu est repris par 25 cm3 d'eau et extrait trois fois par 50 cm3 de dichlorométhane. Les phases organiques réunies sont séchées sur sulfate de magnésium et amenées à sec à 80 ° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane-méthanol (98-2 en volumes) comme éluant. On obtient 4,4 g d'une huile jaune qui est recristallisée dans 50 cm3 d'éthanol bouillant. On isole ainsi 2 g d'{[(indanyl-1 méthyl)-4 pipéridino]-2 éthyl}-2 naphto[1,8-cd]isothiazole dioxyde-1,1 fondant à 127 ° C.

L'(indanyl-1 méthyl)-4 pipéridine peut être préparée selon la méthode décrite par C.GUEREMY et coll., brevet EP 42322.

EXEMPLE 10

On opère comme dans l'exemple 1, à partir de 2,7 g d'(indanyl-1 méthyl)-4 pipéridine, de 3,37 g de (chloro-3 propyl)-2 naphto[1,8-cd]isothiazole dioxyde-1,1 et de 1 g d'hydrogénocarbonate de sodium dans 50 cm3 de diméthylformamide et 50 cm3 de tétrahydrofuranne. Le mélange est chauffé 24 heures à ébullition puis refroidi à une température voisine de 20 ° C. Après évaporation à sec à 80 ° C sous pression réduite (20 mm de mercure; 2,7 KPa), le résidu est repris par 25 cm3 d'eau et extrait deux fois par 75 cm3 de dichlorométhane. Les phases organiques réuni es sont séchées sur sulfate de magnésium et amenées à sec à 80 ° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane- méthanol (98-2 en volumes) comme éluant. On obtient 4,5 g d'une huile jaune. On additionne à cette huile 0,88 g d'acide oxalique dans 30 cm3 d'acétone. On isole ainsi 3,3 g d'{[-(indanyl-1 méthyl)-4 pipéridino]-3 propyl}-2 naphto[1,8-cd]isothiazole dioxyde-1,1 sous forme d'oxalate fondant à 183 ° C.

EXEMPLE 11

On opère comme dans l'exemple 1, à partir de 1 g d'(indanyl-2 méthyl)-4 pipéridine, de 1,2 g de (chloro-2 éthyl)-2 naphto[1,8-cd] isothiazole dioxyde-1,1 et de 0,72 g d'hydrogénocarbonate de sodium dans 25 cm3 de diméthylformamide. Le mélange est chauffé 24 heures à ébullition puis refroidi à une

température voisine de 20° C. Après évaporation à sec à 80° C sous pression réduite (20 mm de mercure; 2,7 KPa), le résidu est repris par 25 cm3 d'eau et extrait deux fois par 50 cm3 de dichlorométhane. Les phases organiques réunies sont séchées sur sulfate de magnésium et amenées à sec à 80° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane-méthanol (99-1 en volumes) comme éluant. On obtient 1 g d'un solide jaune qui est recristallisé dans 40 cm3 d'éthanol bouillant. On isole ainsi 0,95 g d'{[(indanyl-2 méthyl)-4 pipéridino]-2 éthyl}-2 naphto[1,8-cd] isothiazole dioxyde-1,1 fondant à 126° C.

L'(indanyl-2 méthyl)-4 pipéridine peut être préparée selon la méthode décrite par C.GUEREMY, brevet EP 42322.

EXEMPLE 12

On opère comme dans l'exemple 1, à partir de 1 g d'(indènyl-2 méthyl)-4 pipéridine, de 1,2 g de (chloro-2 éthyl)-2 naphto[1,8-cd]isothiazole dioxyde-1,1 et de 0,71 g d'hydrogénocarbonate de sodium dans 25 cm3 de diméthylformamide. Le mélange est chauffé 12 heures à ébullition puis refroidi à une température voisine de 20° C. Après évaporation à sec à 80° C sous pression réduite (20 mm de mercure; 2,7 KPa), le résidu est repris par 25 cm3 d'eau et extrait deux fois par 75 cm3 de dichlorométhane. Les phases organiques réunies sont séchées sur sulfate de magnésium et amenées à sec à 80° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane-méthanol (99-1 en volumes) comme éluant. On obtient 0,75 g d'une huile jaune. On additionne à cette huile 0,15 g d'acide oxalique dans 30 cm3 d'acétone. On isole ainsi 0,9 g d'un solide jaune qui par recristallisation dans 65 cm3 de méthanol fournit 0,5 g d'{[(indènyl-2 méthyl)-4 pipéridino]-2 éthyl}-2 naphto[1,8-cd] isothiazole dioxyde-1,1 sous forme d'oxalate fondant à 176° C.

L'(indènyl-2 méthyl)-4 pipéridine peut être préparée selon la méthode décrite par C. GUEREMY et coll., brevet EP 42322.

EXEMPLE 13

On opère comme dans l'exemple 1, à partir de 2,4 g de [(fluoro-5 indolyl-2) méthylidène]-4 pipéridine, de 2,94 g de (chloro-2 éthyl)-2 naphto[1,8-cd]isothiazole dioxyde-1,1 et de 0,92 g d'hydrogénocarbonate de sodium dans 50 cm3 de tétrahydrofuranne et 25 cm3 de diméthylformamide. Le mélange est chauffé 12 heures à ébullition puis refroidi à une température voisine de 20° C. Après évaporation à sec à 80° C sous pression réduite (20 mm de mercure; 2,7 KPa), le résidu est repris par 100 cm3 d'eau et extrait deux fois par 75 cm3 de dichlorométhane. Les phases organiques réunies sont séchées sur sulfate de magnésium et amenées à sec à 80° C sous pression réduite (20 mm de mercure; 2,7 PKa). Le résidu est purifié par flash-chromathographie sur colonne de silice, sous pression d'azote, à moyenne pression (0,5-1,5 bar) avec un mélange dichlorométhane-méthanol (98-2 en volumes) comme éluant. On obtient 1,4 g d'une meringue blanche qui est recritallisée dans 25 cm3 de dichlorométhane bouillant pour donner 0,9 g de {[((fluoro-5 indolyl-2) méthylidène)-4 pipéridino]-2 éthyl}-2 2H-naphto[1,8-cd] isothiazole dioxyde-1,1 fondant à 160° C.

Le [(fluoro-5 indolyl-2) méthylidène]-4 pipéridine peut être préparée selon la méthode décrite par V.SNIECKUS, Can. J. Chem., 51, 792, (1973).

EXEMPLE 14

On opère comme dans l'exemple 1 à partir de 1,4 g de (chloro-2 éthyl)-1 benzo[cd] indolone-2, de 1,6 g de [(fluoro-5 indolyl-3) méthyl)-4 pipéridine et de 1,5 g de bicarbonate de sodium dans un mélange de 20 cm3 de diméthylformamide et de 15 cm3 de tétrahydrofuranne. Le mélange est chauffé 48 heures à ébullition puis refroidi à une température voisine de 20° C. Après purification par flash-chromathographie sur colonne de silice, sous courant d'azote, à moyenne pression (0,5-1,5 bar) avec de l'acétate d'éthyle comme éluant et recristallisation dans un mélange de 70 cm3 de cyclohexane et de 60 cm3 d'acétate d'éthyle bouillant, on obtient 1 g de {[(( fluoro-5 indolyl-3) méthyl)-4 pipéridino]-2 éthyl}-1 benzo[cd] indolone-2 fondant à 156° C.

La (chloro-2 éthyl)-1 benzo[cd] indolone-2 peut être préparée de la manière suivante : on opère comme dans l'exemple 1 pour la préparation du (chloro-2 éthyl)-2 naphto[1,8-cd] isothiazole dioxyde-1,1, à partir de 1,1 g d'hydrure de sodium à 50 % en dispersion dans l'huile de vaseline, de 1,9 cm3 de bromo-1 chloro-2 éthane et de 4,1 g de benzo[cd] indolone-2 dans 115 cm3 de diméthylformamide. Le mélange est agité 18

heures à une température proche de 20 ° C, sous courant d'argon. Après purification par flash-chromatographie sur colonne de silice, sous courant d'azote, à moyenne pression (0,5-1,5 bar) avec un mélange d'acétate d'éthyle et de cyclohexane (70-30 en volumes) comme éluant , on obtient 1,4 g de (chloro-2 éthyl)-1 benzo[cd] indolone-2 fondant à 136 ° C.

EXEMPLE 15

On opère comme dans l'exemple 1, à partir de 2,85 g de (chloro-2 éthyl)-2 phényl-3 benzisothiazole-1,2 dioxyde-1,1, de 2,15 g de [(fluoro-5 indolyl-3) méthyl)-4 pipéridine et de 2,3 g de bicarbonate de sodium dans un mélange de 30 cm3 de diméthylformamide et de 20 cm3 de tétrahydrofuranne. Le mélange est chauffé 48 heures à ébullition puis refroidi à une température voisine de 20 ° C. Après purification par flash-chromathographie sur colonne de silice, sous courant d'azote, à moyenne pression (0,5-1,5 bar) avec un mélange d'acétate d'éthyle et de cyclohexane (80-20 en volumes) comme éluant, on obtient 2,6 g de {[(( -fluoro-5 indolyl-3) méthyl)-4 pipéridino]-2 éthyl}-2 phényl-3 benzisothiazole-1,2 dioxyde-1,1, qui est transformé en chlorhydrate fondant à 175 ° C.

Le (chloro-2 éthyl)-2 phényl-3 benzisothiazole-1,2 dioxyde-1,1 peut être préparé de la manière suivante : on opère comme dans l'exemple 1 pour la préparation du (chloro-2 éthyl)-2 naphto[1,8-cd] isothiazole dioxyde-1,1, à partir de 0,77 g d'hydrure de sodium à 50% en dispersion dans l'huile de vaseline, de 1,4 cm3 de bromo-1 chloro-2 éthane et de 4 g de phényl-3 benzisothiazole-1,2 dioxyde-1,1 dans 80 cm3 de diméthylformamide. Le mélange est agité 18 heures à une température proche de 20 ° C, sous courant d'azote. Après purification par flash-chromatographie sur colonne de silice, sous courant d'azote, à moyenne pression (0,5-1,5 bar) avec un mélange d'acétate d'éthyle et de cyclohexane (10-90 en volumes) comme éluant , on obtient 3 g de (chloro-2 éthyl)-2 phényl-3 benzisothiazole-1,2 dioxyde-1,1 fondant à 121 ° C.

Le phényl-3 benzisothiazole-1,2 dioxide-1,1 peut être préparé de la manière suivante : à une solution de 80 cm3 d'acide sulfurique concentré refroidie à 0 ° C, on ajoute 10 g de N-tert-butyl (α-hydroxy benzyl)-2 benzènesulfonamide. Le mélange est ensuite agité 1 heure à 25 ° C, puis versé dans 800 cm3 d'eau glacée. Après 1 heure d'agitation, le précipité est essoré puis repris par 100 cm3 de dichlorométhane. La solution organique est lavée à l'eau (2 fois 50 cm3), séchée sur sulfate de magnésium et concentrée à sec sous pression réduite (2,7 kPa). On obtient 7,1 g de phényl-3 benzisothiazole-1,2 dioxide-1,1 fondant à 118 ° C.

Le N-tert-butyl (α-hydroxy benzyl)-2 benzèsulfonamide peut être préparé de la manière suivante : à une solution de 8,5 g de N-tert-butyl benzènesulfonamide dans 100 cm3 de tétrahydrofuranne sec refroidie à 0 ° C, on ajoute 64 cm3 d'une solution de N-butyllithium 1,6 M dans l'hexane. Après une heure d'agitation, on ajoute une solution de 6,5 cm3 de benzaldéhyde dans 30 cm3 de tétrahydrofuranne sec, puis on poursuit l'agitation 2 heures à 0 ° C. Le mélange est traité par 30 cm3 d'acide chlorhydrique 2N et extrait par 100 cm3 d'acétate d'éthyle la solution organique est lavée par 50 cm3 d'eau, séchée sur sulfate de magnésium et concentrée à sec sous pression réduite (2,7 kPa). Le résidu solide est lavé par 50 cm3 d'oxyde d'isopropyle, essoré et séché. On obtient 11,7 g de N-tert-butyl (α-hydroxy benzyl)-2 benzènesulfonamide fondant à 160 ° C.

Le N-tert-butyl benzènesulfonamide peut être préparé par la méthode décrite par J.G. LOMBARDINO, J. Org. Chem., 36, 1843, (1971).

EXEMPLE 16

On opère comme dans l'exemple 1 à partir de 1,9 g de (chloro-2 éthyl)-2 2H-isothiazolo[3,4,5-de] isoquinoléine dioxyde-1,1, de 1,7 g de [(fluoro-5 indolyl-3) méthyl)-4 pipéridine et de 1,8 g de bicarbonate de sodium dans 40 cm3 de diméthylformamide. Le mélange est chauffé 18 heures à ébullition puis refroidi à une température voisine de 20 ° C. Après purification par flash-chromathographie sur colonne de silice, sous courant d'azote, à moyenne pression(0,5-1,5 bar) avec un mélange d'acétate d'éthyle et de méthanol (80-20 en volumes) comme éluant et recristallisation dans 160 cm3 d'acétonitrile bouillant, on obtient 0,52 g de {[(( fluoro-5 indolyl-3) méthyl)-4 pipéridino]-2 éthyl}-2 2H-isothiazolo[3,4,5-de] isoquinoléine dioxyde-1,1 fondant à 207 ° C.

Le (chloro-2 éthyl)-2 2H-isothiazolo[3,4,5-de] isoquino léine dioxyde-1,1 peut être préparé de la manière suivante : à une solution du sel de sodium du 2H-isothiazolo[3,4,5-de] isoquinoléine dioxyde-1,1 (obtenue par chauffage, après cessation du dégagement gazeux, d'une solution de 5,1 g de bromo-4 isoquinolyl-5 sulfonamide dans 30 cm3 de diméthylformamide et d'une suspension de 1,1 g d'hydrure de sodium en dispersion à 80% dans l'huile de vaseline dans 50 cm3 de diméthylformamide, pendant 3 heures à 110 ° C) refroidie à 20 ° C, on ajoute 1,5 cm3 de bromo-2 chloro-1 éthane . Le milieu réactionnel est agité 2 heures

et 30 minutes à 110 °C puis 18 heures à une température proche de 20 °C. Le mélange réactionnel est concentré à sec à 50 °C sous pression réduite (0,5 mm de mercure ; 0,07-kPa). Le résidu est repris par 500 cm3 de dichlorométhane ; la phase organique est lavée par 3 fois 250 cm3 d'eau distillée, décantée, séchée sur du sulfate de magnésium anhydre, filtrée et concentrée à sec (20 mm de mercure ; 2,7kPa). Le résidu obtenu est purifié par flash-chromatographie sur colonne de silice, sous courant d'argon à moyenne pression (0,1-1,5 bar) avec un mélange d'acétate d'éthyle et de cyclohexane (80-20 en volumes) comme éluant. On obtient 1,3 g de (chloro-2 éthyl)-2 2H-isothiazolo[3,4,5-de] isoquinoléine dioxyde-1,1 sous la forme d'un solide brun orangé utilisé à l'état brut dans les synthèses ultérieures.

Le bromo-4 isoquinolyl-5 sulfonamide peut être obtenu de la manière suivante : on fait barbotter jusqu'à saturation de l'ammoniac dans du tétrahydrofuranne sec à -50 °C. Cette solution est traitée par une suspension de 8 g de chlorure de bromo-4 isoquinolyl-5 sulfonyle dans 50 cm3 de tétrahydrofuranne. On laisse revenir le mélange réactionnel progressivement à 20 °C. Le précipité formé est essoré, lavé 3 fois par 10 cm3 de tétrahydrofuranne, 3 fois par 30 cm3 d'eau et 2 fois par 10 cm3 d'acétate d'éthyle puis essoré et séché. On obtient 4,5 g de bromo-4 isoquinolyl-5 sulfonamide sous la forme d'un solide beige dont le point de fusion est supérieur à 270 °C.

Le chlorure de bromo-4 isoquinolyl-5 sulfonyle peut être obtenu de la manière suivante : une solution de 4,46 g d'amino-5 bromo-4 isoquinoléine dans 44 cm3 d'acide chlorhydrique (d = 1,19) est refroidie à -5 °C puis traitée par une solution de 1,93 g de nitrite de sodium dans 10 cm3 d'eau. Le mélange réactionnel est agité 1 heure à 0 °C. La solution obtenue est versée sur une solution saturée de dioxyde de soufre dans 48 cm3 d'acide acétique à laquelle a été ajoutée une solution de 0,65 g de chlorure cuivreux dans 5,6 cm3 d'eau. Le mélange réactionnel est agité jusqu'à la fin du dégagement gazeux puis est extrait 2 fois par 100 cm3 de dichlorométhane. Les phases organiques sont réunies, lavées à l'eau, séchées sur sulfate de magnésium et concentrées. On obtient 5,6 g de chlorure de bromo-4 isoquinolyl-5 sulfonyle sous la forme d'un solide jaune utilisé tel quel dans les synthèses ultérieures.

L'amino-5 bromo-4 isoquinoléine peut être préparée de la manière suivante : à une solution de 90 g de chlorure stanneux dans 100 cm3 d'acide chlorhydrique concentré (d = 1,19), on ajoute progressivement une suspension de 25,3 g de bromo-4 nitro-5 isoquinoléine dans 180 cm3 d'acide chlorhydrique 2N. Le mélange réactionnel est chauffé au reflux pendant 90 minutes puis est refroidi et versé dans 2 litres de solution de soude 2N en agitant et en refroidissant vers 0 °C. Le précipité est lavé à l'eau, essoré et séché. On obtient 21,6 g d'amino-5 bromo-4 isoquinoléine sous la forme de cristaux jaunes fondant à 155 °C.

La bromo-4 nitro-5 isoquinoléine peut-être obtenue par le procédé décrit par M.D. NAIR et coll., Indian J. Chem. Soc., 5, 224 (1967).

EXEMPLE 17

On porte 7 heures au reflux 3,6 g d'un mélange équimoléculaire d'(indanyl-1)-4 pipéridine et d'(indènyl-3)-4 pipéridine, 5,1 g de (chloro-3 propyl)-2 naphto[1,8-cd] isothiazole dioxyde- 1,1 et 1,5 g d'hydrogénocarbonate de sodium dans 50 cm3 de diméthylformamide. Après addition de 50 cm3 d'eau, 200 cm3 de dichlorométhane, séchage de la phase organique sur sulfate de magnésium anhydre et concentration à sec à 80 °C sous pression réduite (20 mm de mercure ; 2,7 kPa), on isole 8,5 g dune huile brune qui est purifiée par flash-chromatographie sur colonne de silice, sous pression d'azote, à moyenne pression (0,5-1,5 bar) avec un mélange dichlorométhane-méthanol (97-3 en volumes) comme éluant. On obtient, d'une part, 1,3 g d'une huile incolore qui dissoute dans 35 cm3 d'acétone et addition d'éther chlorhydrique (4N) conduit à 1,5 g d'{[(indènyl-3)-4 pipéridino]-3 propyl)-2 naphto[1,8-cd] isothiazole dioxyde-1,1 sous forme de chlorhydrate fondant à 220 °C et, d'autre part, 1,6 g d'une huile incolore qui dissoute dans 30 cm3 d'acétone et addition d'éther chlorhydrique (4N) conduit à 1,3 g d'{[(indanyl-1)-4 pipéridino]-3 propyl}-2 naphto[1,8-cd] isothiazole dioxyde-1,1 sous forme de chlorhydrate fondant à 237 °C.

Le mélange équimoléculaire d'(indanyl-1)-4 pipéridine et d'(indènyl-3)-4 pipéridine peut être préparé de la façon suivante : un mélange de 5,7 g d'hydroxy-1 (pyridyl-4)-1 indane, 2,5 cm3 d'acide chlorhydrique concentré (12N), 75 $cm^3$ de méthanol et 0,5 g d'oxyde de platine est hydrogéné sous une pression de 5 bar à une température voisine de 25 °C pendant 5 heures. Après filtration du catalyseur, addition de 20 cm3 d'eau, extraction par 100 cm3 de dichlorométhane, séchage de la phase organique sur sulfate de magnésium anhydre, concentration à sec à 50 °C sous pression réduite (20 mm de mercure : 2,7 kPa) on isole 5 g d'une huile brune qui est purifiée par flash-chromatographie sur silice, sous pression d'azote, à moyenne pression (0,5-1,5 bar), avec un mélange dichlorométhane-méthanol (90-10 en volumes) comme éluant. On obtient ainsi 4 g d'une huile incolore qui est un mélange d'(indanyl-1)-4 pipéridine et d'(indènyl-3)-4 pipéridine dans un rapport équimoléculaire déterminé par RMN du proton (250 MHz) dans le chloroforme deutéré. Cette huile est utilisée telle quelle dans les synthèses ultérieures.

L'hydroxy-1 (pyridyl-4)-1 indane peut être préparé de la façon suivante : à une solution de 7,8 g de bromo-4 pyridine dans 35 cm3 d'éther diéthylique, à - 78 °C, on additionne 40,6 cm3 de n-butyl lithium (1,6 M dans l'hexane). Le milieu réactionnel est laissé 1 heure à - 40 °C. On ajoute ensuite 6,6 g d'indanone-1 dissoute dans 25 cm3 d'éther diéthylique et le mélange est agité 12 heures à une température proche de 25 °C. Après addition de 50 cm3 d'eau, extraction par trois fois 75 cm3 de dichlorométhane, séchage des phases organiques réuni es sur sulfate de magnésium anhydre et concentration à sec à 50 °C sous pression réduite (20 mm de mercure : 2,7 kPa), on isole 11,8 g d'une huile jaune qui purifiée par flash-chromatographie sur silice, sous courant d'azote, à moyenne pression (0,5-1,5 bar), avec un mélange dichlorométhane-méthanol (98-2 en volumes) comme éluant donne 5,7 g d'hydroxy-1 (pyridyl-4) -1 indane fondant à 151 °C.

EXEMPLE 18

On porte 16 heures à 150 °C un mélange de 1 g de (fluoro-8 tétrahydro-1,2,3,4 5H-pyrido[4,3-b] indolyl-2 méthyl)-4 pipéridine, 0,94 g de (chloro-2 éthyl)-2 naphto[1,8-cd] isothiazole dioxyde-1,1 et 0,59 g g d'hydrogénocarbonate de sodium dans 25 cm3 de diméthyl-1,3 imidazolidinone-2. Après retour à une température proche de 25 °C, on verse cette solution dans 75 cm3 d'eau et on extrait par trois fois 75 cm3 d'acétate d'éthyle. Les phases organiques réunies sont séchées sur sulfate de magnésium et amenées à sec à 80 °C sous pression réduite (20 mm de mercure : 2,7 kPa). Le résidu est purifié par flash-chromatographie sur colonne de silice, sous pression d'azote, à moyenne pression (0,5-1,5 bar) avec un mélange dichlorométhane-méthanol (90-10 en volumes) comme éluant. On obtient 0,35 g de {[(fluoro-8 tétrahydro-1,2,3,4 5H-pyrido[4,3-b] indolyl-2 méthyl)-4 pipéridino]-2 éthyl}-2 naphto[1,8-cd] isothiazole dioxyde-1,1 fondant à 228 °C.

La (fluoro-8 tétrahydro-1,2,3,4 5H-pyrido[4,3-b] indolyl-2 méthyl)-4 pipéridine peut être préparée de la façon suivante : 47,3 g de trityl-1 (fluoro-8 tétrahydro-1,2,3,4 5H-pyrido[4,3-b] indolyl-2 méthyl)-4 pipéridine, 225 cm3 d'acide chlorhydrique 5N et 450 cm3 d'éthanol sont agités pendant 12 heures à une température proche de 25 °C. On concentre le tout à sec à 40 °C sous pression réduite (20 mm de mercure : 2,7 kPa). A l'huile ainsi obtenue, on ajoute 270 cm3 d'eau puis on extrait par 50 cm3 d'éther diéthylique. La phase aqueuse est neutralisée par de la soude concentrée (10N) et extraite par deux fois 300 cm3 d'acétate d'éthyle. Les phases organiques réunies sont séchées sur sulfate de magnésium anhydre et concentrées à sec à 50 °C sous pression réduite (20 mm de mercure : 2,7 kPa). On isole ainsi 13,7 g d'un solide jaune que l'on purifie par flash-chromatographie sur colonne de silice, sous pression d'azote, à moyenne pression (0,5-1,5 bar) avec un mélange acétate d'éthyle-méthanol-triéthylamine (70-29-1 en volumes) comme éluant. On obtient 6 g de (fluoro-8 tétrahydro-1,2,3,4 5H-pyrido[4,3-b] indolyl-2 méthyl)-4 pipéridine se présentant sous forme d'une meringue jaune utilisée telle quelle dans les synthèses ultérieures.

La trityl-1 (fluoro-8 tétrahydro-1,2,3,4 5H-pyrido[4,3-b] indolyl-2 méthyl)-4 pipéridine peut être préparée de la façon suivante : un mélange de 17 g de fluoro-8 tétrahydro-1,2,3,4 5H-pyrido[4,3-b] indole, 45,7 g de trityl-1 para-toluènesulfonyloxyméthyl-4 pipéridine, 24,7 g de carbonate de potassium et 225 cm3 de diméthylformamide sont chauffés à 100 °C pendant 5 heures. Après retour à une température voisine de 25 °C, on additionne 90 cm3 d'eau et on extrait par 450 cm3 d'acétate d'éthyle. La phase organique est séchée sur sulfate de magnésium anhydre. Après concentration à sec à 50 °C sous pression réduite (20 mm de mercure : 2,7 kPa) on isole 77,6 g de trityl-1 (fluoro-8 tétrahydro-1,2,3,4 5H-pyrido[4,3-b] indolyl-2 méthyl)-4 pipéridine se présentant sous forme d'une meringue jaune qui est utilisée directement sans autre purification.

La trityl-1 para-toluènesulfonyloxyméthyl-4 pipéridine peut être préparée selon la méthode décrite par C. GUEREMY, brevet EP 42322.

Le fluoro-8 tétrahydro-1,2,3,4 5H-pyrido[4,3-b] indole peut être préparé selon la méthode décrite par J.J. PLATTNER, brevet US 4 001 263.

EXEMPLE 19

On porte 16 heures à 150 °C un mélange de 0,9 g de (quinolyl-3 méthyl)-4 pipéridine, 0,8 g de (chloro-2 éthyl)-2 naph to[1,8-cd] isothiazole dioxyde-1,1, 0,67 g d'hydrogénocarbonate de sodium et 0,6 g d'iodure de sodium dans 25 cm3 de diméthyl-1,3 imidazolidinone-2 . Après retour à une température proche de 25 °C, on verse cette solution dans 75 cm3 d'eau et on extrait par trois fois 75 cm3 d'acétate d'éthyle. Les phases organiques réunies sont séchées sur sulfate de magnésium et amenées à sec à 80 °C sous pression réduite (20 mm de mercure : 2,7 kPa). Le résidu est purifié par flash-chromatographie sur colonne de silice, sous pression d'azote, à moyenne pression (0,5-1,5 bar), avec un mélange dichlorométhane-

méthanol (98-2 en volumes) comme éluant. On obtient 0,52 g d'une huile jaune qui dissoute dans 10 cm3 d'acétone et addition de 0,2 g d'acide oxalique donne 0,55 g de {[(quinolyl-3 méthyl)-4 pipéridino]-2 éthyl}-2 naphto[1,8-cd] isothiazole dioxyde1,1 sous forme d'oxalate fondant à 150 °C.

La (quinolyl-3 méthyl)-4 pipéridine peut être préparée de la façon suivante : un mélange de 5,5 g de benzoyl-1 [(quinolyl-3) carbonyl]-4 pipéridine, 75 cm3 de diéthylèneglycol et 6 g d'hydrazine monohydrate est chauffé à 100 °C pendant 3 heures. On additionne ensuite à cette température, 5 g d'hydroxyde de potassium et la température est amenée à 160 °C pendant 2 heures. Après refroidissement à une température proche de 25 °C, la masse réactionnelle est introduite dans 200 cm3 d'eau. Après extraction par 100 cm3 de dichlorométhane, séchage de la phase organique sur sulfate de magnésium anhydre et concentration, on obtient une huile qui est purifiée par chromatographie sur silice, sous une pression de 10 bar, avec un mélange toluène-diéthylamine-éthanol (80-10-10 en volumes) comme éluant. L'huile incolore (3 g) en présence d'éthanol chlorhydrique donne 1,9 g de (quinolyl-3 méthyl)-4 pipéridine sous forme de chlorhydrate fondant à 265 °C.

La benzoyl-1 [(quinolyl-3) carbonyl]-4 pipéridine peut être préparée de la façon suivante : à une solution de 33,28 g de bromo-3 quinoléine dans 320 cm3 d'éther diéthylique, on additionne à - 78 °C, en 30 minutes, 100 cm3 de n-butyl lithium (1,6 M dans l'hexane), puis 41,7 g de benzoyl-1 isonipécotate d'éthyle dissous dans 320 cm3 de tétrahydrofuranne. Après retour à une température proche de 25 °C, on laisse le tout 1 heure à cette température et on additionne ensuite 30 cm3 d'eau. Après extraction par 100 cm3 d'acétate d'éthyle, séchage de la phase organique sur sulfate de magnésium anhydre et concentration, on obtient une huile qui est purifiée par chromatographie sur silice, sous une pression de 10 bar, avec de l'acétate d'éthyle comme éluant. On isole ainsi 10,3 g de benzoyl-1 [(quinolyl-3) carbonyl]-4 pipéridine fondant à 170 °C.

Le benzoyl-1 isonipécotate d'éthyle a été obtenu par benzoylation de l'isonipécotate d'éthyle par le chlorure de benzoyle.

EXEMPLE 20

On porte 16 heures à 150 °C un mélange de 1,8 g de (chromanyl-3 méthyl)-4 pipéridine, 1,8 g de (chloro-2 éthyl)-2 naphto[1,8-cd] isothiazole dioxyde-1,1, 1,68 g d'hydrogénocarbonate de sodium et 1 g d'iodure de sodium dans 25 cm3 de diméthyl-1,3 imidazolidinone-2. Après retour à une température proche de 25 °C, on verse cette solution dans 75 cm3 d'eau et on extrait par trois fois 75 cm3 d'acétate d'éthyle. Les phases organiques réunies sont séchées sur sulfate de magnésium et amenées à sec à 80 °C sous pression réduite (20 mm de mercure : 2,7 kPa). Le résidu est purifié par flash-chromatographie sur colonne de silice, sous pression d'azote, à moyenne pression (0,5-1,5 bar) avec un mélange dichlorométhane-méthanol (99-1 en volumes) comme éluant. On obtient 1,9 g d'une huile jaune qui dissoute dans 30 cm3 d'acétone et addition de 0,37 g d'acide oxalique donne 1,6 g d'un solide jaune qui est recristallisé dans 20 cm3 de diméthylformamide bouillant conduisant à 1,4 g de {[(chromanyl-3 méthyl)-4 pipéridino]-2 éthyl}-2 naphto[1,8-cd] isothiazole dioxyde-1,1 sous forme d'oxalate fondant à 230 °C.

La (chromanyl-3 méthyl)-4 pipéridine peut être préparée de la façon suivante : une solution de 14 g de (pyridyl-4) méthylène-3 chromanone-4 dans 140 cm3 d'acide acétique est hydrogénée en présence de palladium sur charbon (10 %), à une température proche de 25 °C et sous pression atmosphérique, pendant 5 heures. On obtient 13 g de (chromanyl-3 méthyl)-4 pipéridine se présentant sous forme d'une huile jaune qui est utilisée sans autre purification.

La (pyridyl-4) méthylène-3 chromanone-4 peut être préparée sde la façon suivante : un mélange de 30 g de chromanone-4, 42,8 g de (pyridyl-4) carboxaldéhyde, 30 cm3 de soude concentrée (10N), 100 cm3 de méthanol et 60 cm3 d'eau est agité pendant 2 heures à une température proche de 10 °C. On isole ainsi directement 9,2 g de (pyridyl-4) méthylène-3 chromanone-4 fondant à 132 °C.

EXEMPLE 21

On porte 16 heures à 150 °C un mélange de 1,4 g de (tétrahydro1,2,3,4 quinolyl-3 méthyl)-4 pipéridine, 1,4 g de (chloro-2 éthyl) -2 naphto[1,8-cd] isothiazole dioxyde-1,1, 1,3 g d'hydrogénocarbonate de sodium et 0,78 g d'iodure de sodium dans 25 cm3 de diméthyl-1,3 imidazolidinone-2. Après retour a une température proche de 25 °C, on verse cette solution dans 75 cm3 d'eau et on extrait par trois fois 75 cm3 d'acétate d'éthyle. Les phases organiques réunies sont séchées sur sulfate de magnésium et amenées à sec à 80 °C sous pression réduite (20 mm de mercure : 2,7 kPa). Le résidu est purifié par flash-chromatographie sur colonne de silice, sous pression d'azote, à moyenne pression (0,5-1,5 bar) avec un mélange dichlorométhane-méthanol (99-1 en volumes) comme éluant. On obtient 2,3 g d'une huile jaune

qui après dissolution dans 30 cm3 d'acétone et addition de 0,47 g d'acide oxalique donne 0,85 g d'un solide jaune. Ce solide est recristallisé dans 15 cm3 de diméthylformamide bouillant et conduit à 0,53 g de {[(tétrahydro-1,2,3,4 quinolyl-3 méthyl)-4 pipéridino]-2 éthyl}-2 naphto[1,8-cd] isothiazole dioxyde-1,1 sous forme d'oxalate fondant à 134 °C.

La (tétrahydro-1,2,3,4 quinolyl-3 méthyl)-4 pipéridine peut être préparée de la façon suivante : à 8,25 g d'acétyl-1 (pipéridyl-4 méthyl)-3 tétrahydro-1,2,3,4 quinolone-4 dans 25 cm3 de diéthylèneglycol, on ajoute 5,1 cm3 d'hydrazine monohydrate et on porte à 150 °C pendant 20 minutes. On laisse ensuite la température revenir à 110 °C, ajoute 4,9 g d'hydroxyde de potassium en pastilles et chauffe à 195 °C pendant 1 heure. Après refroidissement à une température proche de 50 °C, on additionne 200 cm3 d'eau et on extrait par trois fois 100 cm3 d'éther diéthylique. Les phases organiques réunies sont séchées sur sulfate de magnésium anhydre et concentrées à sec à 40 °C sous pression réduite (20 mm de mercure : 2,7 kPa). On isole 3,6 g d'une laque jaune qui dissoute dans 18 cm3 d'éthanol et addition de 2,2 cm3 d'éther chlorhydrique 7N donne 2,5 g de (tétrahydro-1,2,3,4 quinolyl-3 méthyl)-4 pipéridine sous forme de chlorhydrate fondant à 220 °C.

L'acétyl-1 (pipéridyl-4 méthyl)-3 tétrahydro-1,2,3,4 quinolone-4 peut être préparé de la façon suivante : 9,3 g de (pyridyl-4 méthylène)-3 acétyl-1 tétrahydro-1,2,3,4 quinolone-4 dissous dans 95 cm3 d'acide acétique sont hydrogénés en présence de 0,93 g d'oxyde de platine, sous pression atmosphérique, pendant 1 heure. On obtient 8,25 g du composé désiré se présentant sous forme d'une laque jaune utilisée telle quelle dans les synthèses ultérieures.

La (pyridyl-4 méthylène)-3 acétyl-1 tétrahydro-1,2,3,4 quinolone-4 peut être préparée de la façon suivante : 15,4 g d'acétyl-1 tétrahydro-1,2,3,4 quinolone-4 sont introduits dans une solution de 25 cm3 d'eau, 40 cm3 de méthanol et 12,5 cm3 de soude 2N. On y additionne, à 0 °C, 17,5 g de (pyridyl-4) carboxaldéhyde. Après 12 heures à 0 °C, le précipité formé est filtré et on isole ainsi 6,8 g de (pyridyl-4 méthylène)-3 acétyl-1 tétrahydro-1,2,3,4 quinolone-4 fondant à 170 °C.

L'acétyl-1 tétrahydro-1,2,3,4 quinolone-4 de 4 g peut être préparée de la manière suivante : un mélange de 4 g de tétrahydro-1,2,3,4 quinolone-4 et 12 cm3 d'anhydride acétique est chauffé au reflux pendant 1 heure. On isole, après neutralisation par de la soude concentrée (10N), extraction à l'éther diéthylique, séchage sur sulfate de magnésium anhydre et concentration, 4,2 g d'un solide jaune fondant à 89 °C.

La tétrahydro-1,2,3,4 quinolone-4 peut être préparée selon la méthode décrite par R. C. ELDERFIELD, J. Am. Chem. Soc., 71, 1901 (1949).

EXEMPLE 22

On porte 16 heures à 150 °C un mélange de 1,3 g de [(fluoro-7 tétrahydro-1,2,3,4 naphtyl)-2 méthyl]-4 pipéridine, 1,22 g de (chloro-2 éthyl)-2 naphto[1,8-cd] isothiazole dioxyde-1,1, 1,15 g d'hydrogénocarbonate de sodium et 0,69 g d'iodure de sodium dans 25 cm3 de diméthyl-1,3 imidazolidinone-2. Après retour à une température proche de 25 °C, on verse cette solution dans 75 cm3 d'eau et on extrait par trois fois 75 cm3 d'acétate d'éthyle. Les phases organiques réunies sont séchées sur sulfate de magnésium et amenées à sec à 80 °C sous pression réduite (20 mm de mercure : 2,7 kPa). Le résidu est purifié par flash-chromatographie sur colonne de silice, sous pression d'azote, à moyenne pression (0,5-1,5 bar) avec un mélange dichlorométhane-méthanol (99-1 en volumes) comme éluant. On obtient 1,7 g d'une huile brune qui dissoute dans 10 cm3 d'acétone et addition de 0,41 g d'acide oxalique donne 1,3 g d'un solide jaune. Ce solide est recristallisé dans 13 cm3 de diméthylformamide bouillant et conduit à 1,05 g de {[(fluoro-7 tétrahydro-1,2,3,4 naphtyl)-2 méthyl)-4 pipéridino]-2 éthyl}-2 naphto[1,8-cd] isothiazole dioxyde-1,1 sous forme d'oxalate fondant à 265 °C.

La [(fluoro-7 tétrahydro-1,2,3,4 naphtyl)-2 méthyl]-4 pipéridine est obtenue par chauffage à 160 °C, pendant 15 minutes, d'un mélange de 13,1 g de [(fluoro-7 tétrahydro-1,2,3,4 oxo-1 naphtyl)-2 méthyl]-4 pipéridine, 9,4 g d'hydrazine monohydrate dans 75 cm3 de diéthylèneglycol. On laisse ensuite la température revenir à 120 °C, ajoute 8,4 g d'hydroxyde de potassium et chauffe le tout à 160 °C pendant 5 heures. Après retour à une température proche de 25 °C, on additionne 650 cm3 d'eau et on extrait par 350 cm3 de chloroforme. Les phases organiques réunies sont séchées sur sulfate de magnésium anhydre et concentrées à sec à 50 °C sous pression réduite (20 mm de mercure : 2,7 kPa). On isole ainsi 14,5 g d'une huile jaune qui est purifiée par chromatographie sur silice avec un mélange toluène-diéthylamine (4-1 en volumes) comme éluant. On isole 3,78 g d'une huile incolore qui dissoute dans 20 cm3 d'éther diéthylique cristallise avec 2 cm3 d'une solution d'acide chlorhydrique éthanolique à 10,3N et conduit à la formation de 3,1 g de [fluoro-7 tétrahydro-1,2,3,4 naphtyl)-2 méthyl]-4 pipéridine sous forme de chlorhydrate fondant à 208 °C.

La [(fluoro-7 tétralone-1)-2 méthyl]-4 pipéridine peut être préparée de la façon suivante : 15,9 g de

fluoro-7 (pyridyl-4 méthylène)-2 tétralone-1 dans 230 cm3 d'acide acétique en présence de 1,59 g d'oxyde de platine sont hydrogénés, sous pression atmosphérique, à une température de 35° C, pendant 6 heures. On obtient ainsi 12,6 g du composé attendu fondant à 224° C sous forme de chlorhydrate.

La fluoro-7 (pyridyl-4 méthylène)-2 tétralone-1 peut être préparée de la façon suivante : un mélange de 75 g de fluoro-7 (pyridyl-4) hydroxyméthyl-2 tétralone-1, 830 cm3 d'acide chlorhydrique concentré (10N) et 270 cm3 d'acide formique à 98 % est chauffé au reflux pendant 3 heures. Après neutralisation par de la soude concentrée (10N), extraction au dichlorométhane et concentration, on isole 67,8 g d'un solide jaune qui recristallisé dans un mélange de 600 cm3 d'éthanol et de 230 cm3 de méthanol donne 47,5 g du composé désiré fondant à 144° C.

Le fluoro-7 (pyridyl-4) hydroxyméthyl-2 tétralone-1 naphtalène peut être préparé de la façon suivante : à un mélange de 45,4 g de fluoro-7 tétralone-1, 140 cm3 de méthanol et 41,5 $cm^3$ de soude 2N, à une température de 5° C, on additionne 59,2 g de (pyridyl-4) carboxaldéhyde. Le tout est agité à une température proche de 25° C pendant 3 heures. On isole directement 68,8 g du composé attendu fondant à 133° C.

La fluoro-7 tétralone-1 peut être préparée selon la méthode décrite par G.A. THIAULT, Bull. Soc. Chim. France, 1308 (1965).

EXEMPLE 23

On porte 16 heures à 150° C un mélange de 2 g d'{(indo lyl-1)-2 éthyl}-4 pipéridine, 2 g de (chloro-2 éthyl)-2 naphto[1,8-cd] isothiazole dioxyde-1,1, 1,26 g d'hydrogénocarbonate de sodium et 1 g d'iodure de sodium dans 25 cm3 de diméthyl-1,3 imidazolidinone-2. Après retour à une température proche de 25° C, on verse cette solution dans 75 cm3 d'eau et on extrait par trois fois 75 cm3 d'acétate d'éthyle. Les phases organiques réunies sont séchées sur sulfate de magnésium et amenées à sec à 80° C sous pression réduite (20 mm de mercure : 2,7 kPa). Le résidu est purifié par flash-chromatographie sur colonne de silice, sous pression d'azote, à moyenne pression (0,5-1,5 bar) avec un mélange dichlorométhane-méthanol (99-1 en volumes) comme éluant. On obtient 3,3 g d'une huile jaune qui après cristallisation dans 30 cm3 d'acétonitrile bouillant fournit 1,52 g d'{{[(indolyl-1)-2 éthyl]-4 pipéridino}-2 éthyl)-2 naphto[1,8-cd] isothiazole dioxyde-1,1 fondant à 152° C.

L'{(indolyl-1)-2 éthyl}-4 pipéridine peut être préparée de la façon suivante : une solution de 37 g de [-(pyridyl-4)-2 éthyl]-1 indole, 3,7 g d'oxyde de platine dans 350 cm3 d'acide acétique est hydrogénée, sous pression atmosphérique, à une température proche de 25° C pendant 10 heures et conduit à 33 g d'{(-indolyl-1)-2 éthyl}-4 pipéridine dont le chlorhydrate fond à 204° C.

Le [(pyridyl-4)-2 éthyl]-1 indole peut être préparé selon la méthode décrite par A.P. GRAY, J. Am. Chem. Soc. 79, 3554 (1957).

EXEMPLE 24

A une solution de 4,6 g d'indène dans 100 cm3 de tétrahydrofuranne, à une température voisine de -78° C, on introduit 25 cm3 de n-butyl lithium (1,6 M) et on laisse le tout 45 minutes à cette température. On additionne ensuite goutte à goutte 13,7 g d'[(oxo-4 pipéridino)-3 propyl]-2 naphto[1,8-cd] isothiazole dioxyde-1,1 dissous dans 80 cm3 de tétrahydrofuranne et on laisse sous agitation pendant 12 heures à une température voisine de 25° C. Après addition de 160 cm3 d'eau, extraction par 500 cm3 de dichlorométhane, séchage de la phase organique sur sulfate de magnésium anhydre et concentra tion à sec sous pression réduite à 50° C (20 mm de mercure : 2,7 kPa), on isole 18 g d'une huile que l'on purifie par flash-chromatographie sur colonne de silice, sous courant d'azote, à moyenne pression (0,5-1,5 bar) avec un mélange dichlorométhane-méthanol (98-2 en volumes) comme éluant. On isole ainsi 5 g d'une meringue jaune qui dissoute dans 100 cm3 d'éther diéthylique et traitée par de l'éther chlorhydrique 4N fournit 4,4 g d'{[(indénylidène-1)-4 pipéridino]-3 propyl}-2 naphto[1,8-cd] isothiazole dioxyde-1,1 sous forme de chlorhydrate fondant à 210° C.

L'[(oxo-4 pipéridino)-3 propyl]-2 naphto[1,8-cd] isothiazole dioxyde-1,1 peut être préparé de la façon suivante : un mélange de 21,5 g de pipéridone-4 monohydrate, 39,4 g de (chloro-3 propyl)-2 naphto[1,8-cd] isothiazole dioxyde-1,1 et 23,5 g d'hydrogénocarbonate de sodium dans 420 cm3 de diméthylformamide et 420 $cm^3$ de tétrahydrofuranne est porté au reflux pendant 16 heures. Après retour à une température proche de 25° C, filtration du précipité formé, addition de 150 cm3 d'eau, extraction par 250 cm3 de dichlorométhane, séchage de la phase organique sur sulfate de magnésium anhydre et concentration à sec à 40° C sous pression réduite (20 mm de mercure : 2,7 kPa), on isole 48,6 g d'une huile jaune que l'on purifie par flash-chromatographie sur colonne de silice, sous courant d'azote, à moyenne pression (0,5-1,5

bar) avec un mélange dichlorométhane-méthanol (99-1 en volumes) comme éluant. On obtient ainsi 36,2 g d'[(oxo-4 piperidino)-3 propyl]-2 naphto [1,8-cd] isothiazole dioxyde-1,1 fondant à 108 ° C.

EXEMPLE 25

Un mélange de 5,3 g de (chloro-2 éthyl)-2 naphto [1,8-cd] isothiazole dioxyde-1,1, 4,6 g de [(fluoro-5 indolyl-3) méthyl]-4 pipéridine et 1,68 g de bicarbonate de sodium dans 120 cm3 de tétrahydrofuranne et 120 cm3 de diméthylformamide est chauffé 48 heures à ébullition puis refroidi à une température voisine de 20 ° C. Le précipité formé est séparé par filtration et le filtrat est évaporé à sec à 40 ° C sous pression réduite (20 mm de mercure ; 2,7KPa). Le résidu obtenu est purifié par flash-chromatographie sur colonne de silice, sous courant d'argon, à moyenne pression (0,5 -1,5 bar) avec de l'acétate d'éthyle comme éluant. On obtient 4,4 g d'une huile brune. 2g de cette huile sont recristallisés dans 15 cm3 d'acétonitrile bouillant. On obtient ainsi 1,6 g de {[((fluoro-5 indolyl-3) méthyl)-4 pipéridino]-2 éthyl]-2 naphto[1,8-cd] isothiazole dioxyde-1,1 fondant à 161 ° C.

EXEMPLE 26

On porte 12 heures à reflux un mélange de 2,9 g de [(fluoro-5 indolyl-2) méthyl]-4 pipéridine, 3,5 g de (chloro-2 éthyl-2 naphto[1,8-cd] isothiazole dioxyde-1,1 et 1,1 g d'hydrogénocarbonate de sodium dans 30 cm3 de tétrahydrofuranne et 30 cm3 de diméthylformamide. Après évaporation à sec à 80 ° C sous pression réduite (20 mm de mercure ; 2,7 kPa), le résidu est repris par 150 cm3 d'eau et extrait par deux fois 75 cm3 d'acétate d'éthyle. Les phases organiques réunies sont séchées sur sulfate de magnésium et amenées à sec à 80 ° C sous pression réduite (20 mmm de mercure ; 2,7kPa). Le résidu est purifié par flash-chromatographie sur colonne de silice, sous courant d'azote, à moyenne pression (0,5 - 1,5 bar) avec un mélange cyclohexane-acétate d'éthyle (50 - 50 en volumes) comme éluant. On obtient 2,3 g de {[(-(fluoro-5 indolyl-2) méthyl)-4 pipéridino-2] éthyl}-2 naphto[1,8-cd] isothiazole dioxyde-1,1 fondant à 154 ° C.

La [(fluoro-5 indolyl-2) méthyl]-4 pipéridine peut être préparée selon la méthode décrite par V. SHIECKUS, Can. J. Chem., 51, 792 (1973).

EXEMPLE 27

On porte 12 heures à reflux un mélange de 0,4 g d'(indolyl-3 méthyl)-4 pipéridine, 0,53 g de (chloro-2 éthyl)-2 naphto[1,8-cd] isothiazole dioxyde-1,1, 0,17 g d'hydrogénocarbonate de sodium et 10 cm3 de diméthylformamide. Après évaporation à sec à 80 ° C sous pression réduite (20 mm de mercure ; 2,7 kPa), le résidu est repris par 30 cm3 d'eau et extrait par deux fois 50 cm3 d'acétate d'éthyle. Les phases organiques réunies sont séchées sur sulfate de magnésium et amenées à sec à 80 ° C sous pression réduite (200 mm de mercure ; 2,7 kPa). Le résidu est purifié par flash-chromatographie sur colonne de silice, sous courant d'azote, à moyenne pression (0,5 -1,5 bar) avec un mélange dichlorométhane-méthanol (99 -1 en volumes) comme éluant. On obtient 0,4 g d'{[(indolyl-3 méthyl)-4 pipéridino]-2 éthyl}-2 naphto-[1,8-cd] isothiazole dioxyde-1,1 fondant à 184 ° C.

L'(indolyl-3 méthyl)-4 pipéridine peut être préparée selon la méthode décrite par C. GUEREMY, J. Med. Chem., 23, 1306 (1980).

EXEMPLE 28

On opère comme dans l'exemple 25 à partir de 4,4g de (chloro-2 éthyl)-1 dihydro-5,6 1H,4H-1,2,5-thiadiazolo [4,3,2-ij] quinoléine dioxyde-2,2, de 3,8 g de [(fluoro-5 indolyl-3)méthyl]-4 pipéridine et de 4g de bicarbonate de sodium dans un mélange de 50cm3 de diméthylformamide et de 30 cm3 de tétrahydrofuranne. Le mélange est chauffé 36 heures à ébullition puis refroidi à une température voisine de 20 ° C. Après purification par flash-chromatographie sur colonne de silice, sous courant d'azote, à moyenne pression (0,5 - 1,5 bar) avec de l'acétate d'éthyle comme éluant et recristallisation dans 50 cm3 d'acétonitrile bouillant, on obtient 1 g de {[((fluoro-5 indolyl-3) méthyl)-4 pipéridino]-2 éthyl}-1 dihydro-5,6 1H,4H-1,2,5-thiadiazolo[4,3,2-ij] quinoléine dioxyde-2,2 fondant à 149 ° C.

Le (chloro-2 éthyl)-1 dihydro-5,6 1H,4H-1,2,5-thiadiazolo[4,3,2-ij] quinoléine dioxyde-2,2 peut être préparé de la manière suivante : On opère comme à l'exemple 1 pour la préparation du (chloro-2 éthyl)-2 naphto-[1,8-cd] isothiazole dioxyde-1,1, à partir de 0,82 g d'hydrure de sodium à 50% en dispersion dans l'huile de vaseline, de 1,4 cm3 de bromo-1 chloro-2 éthane et de 3,6 g de dihydro-5,6 1H,4H-1,2,5-thiadiazolo[4,3,2-ij] quinoléine dioxyde-2,2 dans 70 cm3 de diméthylformamide. Le mélange est agité 48 heures à une

température proche de 20 °C, sous courant d'argon. On obtient, après traitement habituel, 4,4 g de (chloro-2 éthyl)-1 dihydro-5,6 1H,4H-1,2,5-thiadiazolo[4,3,2-ij] quinoléine dioxyde-2,2 sous forme d'une huile brune utilisée à l'état brut dans les synthèses ultérieures.

La dihydro-5,6 1H,4H-1,2,5-thiadiazolo[4,3,2-ij] quinoléine dioxyde-2,2 peut être préparée de la manière suivante : à une solution de 9 g d'amino-8 tétrahydro-1,2,5,6 quinoléine dans 90 cm3 de diglyme, on ajoute 6 g de sulfamide. Le mélange est chauffé à 160 °C pendant 90 minutes puis refroidi et dilué dans un mélange de 300 cm3 d'eau et 300 cm3 d'acétate d'éthyle. La phase organique est lavée par 3 fois 300 cm3 d'eau, séchée sur sulfate de magnésium et concentrée à sec sous pression réduite (2,7 KPa). Le résidu est chromatographié sur une colonne de silice (granulométrie 0,2-0,063 mm, diamètre 6 cm, hauteur 60 cm) en éluant, sous une pression de 0,7 bar d'azote, par un mélange de cyclohexane et d'acétate d'éthyle (80-20 en volumes) et en recueillant des fractions de 125 cm3. Les fractions 12 à 22 sont réunies et concentrées à sec sous pression réduite (2,7 KPa). On obtient ainsi 9,4 g de dihydro-5,6 1H,4H-1,2,5-thiadiazolo[4,3,2-ij] quinoléine dioxyde-2,2 fondant à 96 °C.

L'amino-8 tétrahydro-1,2,5,6 quinoléine peut être préparée selon la méthode décrite par HAZLEWOOD et coll., J. Pr. Soc., N. S. WALES, 71, 462 (1937-1938).

EXEMPLE 29

On opère comme dans l'exemple 25 à partir de 4,1 g de (chloro-2 éthyl)-2 naphto [1,8-cd] isothiazole dioxyde-1,1, de 3,6 g de [(fluoro-6 indolyl-3) méthyl]-4 pipéridine et de 1,3 g de bicarbonate de sodium dans un mélange de 100 cm3 de diméthylformamide et de 75 cm3 de tétrahydrofuranne. Le mélange est chauffé 8 heures à ébullition puis 48 heures à une température voisine de 20 °. Après purification par flash-chromatographie sur colonne de silice, sous courant d'azote, à moyenne pression (0,5-1,5 bar) avec du dichlorométhane puis de l'acétate d'éthyle comme éluants et recristallisation dans 40 cm3 d'acétonitrile bouillant, son obtient 1,2 g de {[((fluoro-6 indolyl-3) méthyl)-4 pipéridino]-2 éthyl}-2 naphto [1,8-cd] isothiazole dioxyde-1,1 fondant à 177 °C.

La [(fluoro-6 indolyl-3) méthyl]-4 pipéridine peut être préparée selon la méthode décrite par C. GUEREMY et coll., J. Med. Chem., 23, 1306, (1980).

EXEMPLE 30

On opère comme dans l'exemple 25 à partir de 1,3 g (chloro-2 éthyl)-2 naphto [1,8-cd] isothiazole dioxyde-1,1, de 1,3g de chlorhydrate de [(fluoro-4 indolyl-3) méthyl]-4 pipéridine et de 1,3 g de bicarbonate de sodium dans un mélange de 15 cm3 de diméthylformamide et de 10 cm3 de tétrahydrofuranne. Le mélange est chauffé 48 heures à ébullition puis refroidi à une température voisine de 20 °C. Après purification par flash-chromatographie sur colonne de silice, sous courant d'azote, à moyenne pression (0,5-1,5 bar) avec de l'acétate d'éthyle comme éluant et recristallisation dans 200 cm3 d'oxyde d'isopropyle bouillant, on obtient 0,8 g de {[((fluoro-4 indolyl-3) méthyl)-4 pipéridino]-2 éthyl}-2 naphto [1,8-cd] isothiazole dioxyde-1,1 fondant à 123 °C.

La [(fluoro-4 indolyl-3)-2 méthyl]-4 pipéridine peut être préparée selon la méthode décrite par R.T. BORCHARDT et coll., J. Het. Chem., 24, 1499 (1987).

EXEMPLE 31

On opère code dans l'exemple 25 à partir de 6,9 g de (chloro-2 éthyl)-1 oxo-2 tétrahydro-1,2,5,6 4H-imidazo [4,5,1-ij] quinoléine, de 6,8 g de [(fluoro-5 indolyl-3) méthyl)-4 pipéridine et de 7,3 g de bicarbonate de sodium dans un mélange de 100 cm3 de diméthylformamide et de 60 cm3 de tétrahydrofuranne. Le mélange est chauffé 36 heures à ébullition puis refroidi à une température voisine de 20 °C. Après purification par flash-chromatographie sur colonne de silice, sous courant d'azote, à moyenne pression (0,5-1,5 bar) avec un mélange d'acétate d'éthyle et de méthanol (90-10 en volumes) comme éluant et recristallisation dans 150 cm3 d'acétonitrile bouillant, on obtient 3,5 g de {[(fluoro-5 indolyl-3) méthyl-4 pipéridino]-2 éthyl)-1 oxo-2 tétrahydro-1,2,5,6 4H-imidazo [4,5,1-ij] quinoléine fondant à 177 °C.

La (chloro-2 éthyl)-1 oxo-2 tétrahydro-1,2,5,6 4H-imidazo [4,5,1-ij] quinoléine peut être préparée de la manière suivante : on opère comme à l'exemple 1 pour la préparation du (chloro-2 éthyl)-2 naphto [1,8-cd] isothiazole dioxyde-1,1, à partir de 2,54 g d'hydrure de sodium à 50% en dispersion dans l'huile de vaseline, de 4,4 cm3 de bromo-1 chloro-2 éthane et de 9,3 g d'oxo-2 tétrahydro-1,2,5,6 4H-imidazo [4,5,1-ij] quinoléine dans 250 cm3 de diméthylformamide. Le mélange est agité 48 heures à une température proche de 20 °C, sous courant d'argon. Après purification par flash-chromatographie sur colonne de silice, sous

courant d'argon, à moyenne pression (0,5-1,5 bar) avec de l'acétate d'éthyle comme éluant, on obtient 6,9 g de (chloro-2 éthyl)-1 oxo-2 tétrahydro-1,2,5,6 4H-imidazo [4,5,1-ij] quinoléine sous forme d'une huile utilisée à l'état brut dans les synthèse ultérieures.

L'oxo-2 tétrahydro-1,2,5,6 4H-imidazo [4,5,1-ij] quinoléine peut être préparée de la manière suivante : on opère comme à l'exemple 28 pour la préparation de la dihydro-5,6 1H, 4H-1,2,5-thiadiazolo [4,3,2-ij] quinoléine dioxyde-2,2, mais à partir de 12,6 g d'amino-8 tétrahydro-1,2,5,6 quinoléine et 6,5 g d'urée dans 150 cm3 de diglyme. Le mélange est chauffé à 160° C pendant 4 heures ; après traitement habituel et purification par flash-chromatographie sur colonne de silice, sous courant d'argon, à moyenne pression (0,5-1,5 bar), avec de l'acétate d'éthyle code éluant, on obtient 8,4 g d'oxo-2 tétrahydro-1,2,5,6 4H-imidazo [4,5,1-ij] quinoléine fondant à 216° C.

L'amino-8 tétrahydro-1,2,5,6 quinoléine peut être préparée selon la méthode décrite par HAZLEWOOD et coll.,J. Pr. Soc., N.S. WALES, 71, 462 (1937-1938).

## EXEMPLE 32

A une solution agitée de 3,4 g d'hydroxy-3 {[((fluoro-5 indolyl-3) méthyl)-4 piperidino]-2 éthyl}-2 isoindolinone-(RS) dans 21 cm3 d'acide acétique, on ajoute, à une température voisine de 20° C, 2,97 g de zinc en poudre. Le mélange est chauffé 9 heures à ébullition puis refroidi à une température proche de 20° C. Après addition de 30 cm3 d'eau distillée, la solution est alcalinisée à pH 7-8 avec du bicarbonate de sodium. La phase organique est extraite par 4 fois 30 cm3 de dichlorométhane, lavée par 30 cm3 d'eau distillée, séchée sur sulfate de magnésium anhydre, traitée au noir végétal, filtrée et concentrée à sec à 40° C sous pression réduite (20mm de mercure ; 2,7kPa). Le résidu obtenu est purifié par flash-chromatographie sur colonne de silice, sous courant d'argon à moyenne pression (0,5-1,5 bar) avec de l'acétate d'éthyle puis un mélange d'acétate d'éthyle et de méthanol (98-2 en volumes) comme éluants. Après recristallisation dans 40 cm3 d'acétate d'éthyle, on obtient 0,54 g de [[((fluoro-5 indolyl-3) méthyl)-4 pipéridino]-2 éthyl]-2 isoindolinone fondant à 175° C.

L'hydroxy-3 {[((fluoro-5 indolyl-3) méthyl)-4 pipéridino]-2 éthyl}-2 isoindolinone-(RS) peut être préparée de la manière suivante: on opère comme dans l'exemple 25 à partir de 5,5 g de (bromo-2 éthyl)-2 hydroxy-3 isoindolinone-(RS), de 5 g de [(fluoro-5 indolyl-3) méthyl)-4 pipéridine et de 1,8 g de bicarbonate de sodium dans un mélange de 100 cm3 de diméthylformamide et de 125 cm3 de tétrahydrofuranne. Le mélange est chauffé 10 heures à ébullition puis refroidi à une température voisine de 20° C . Après purification par flash-chromatographie sur colonne de silice, sous courant d'azote, à moyenne pression (0,5-1,5 bar) avec de l'acétate d'éthyle puis un mélange acétate d'éthyle-méthanol (98-2 en volumes) comme éluants, on obtient 5,4 g d'hydroxy-3 {[(( fluoro-5 indolyl-3) méthyl)-4 pipéridino]-2 éthyl)-2 isoindolinone-(RS) fondant à 183° C.

La (bromo-2 éthyl}-2 hydroxy-3 isoindolinone-(RS) peut être préparée de la manière suivante: à une solution de 50 g de (bromo-2 éthyl)-2 phtalimide dans 420 cm3 de méthanol et 42 cm3 d'eau distillée, on ajoute, à une température voisine de 20° C, par petites portions 10,5 g de borohydrure de potassium. L'agitation est maitenue 15 heures à une température proche de 20° C, puis le milieu réactionnel est repris par 250 cm3 d'eau distillée. La phase organique est extraite par 4 fois 100 cm3 de dichlorométhane, lavée par 100 cm3 d'eau distillée, séchée sur sulfate de magnésium anhydre, traitée au noir végétal, filtrée et concentrée à sec à 40° C sous pression réduite (20mm de mercure; 2,7kPa). Le résidu obtenu est recristallisé dans 70 cm3 de méthyléthylcétone. On obtient 17,3 g de (bromo-2 éthyl)-2 hydroxy-3 isoindolinone-(RS) fondant à 132° C.

## EXEMPLE 33

On opère comme dans l'exemple 25 à partir de 2,4 g de (bromo-2 éthyl)-2 méthoxy-3 isoindolinone-(RS), de 2,1 g de [(fluoro-5 indolyl-3) méthyl)-4 pipéridine et de 0,74 g de bicarbonate de sodium dans un mélange de 50 cm3 de diméthylformamide et de 40 cm3 de tétrahydrofuranne. Le mélange est chauffé 10 heures à ébullition puis refroidi à une température voisine de 20° C. Après purification par flash-chromatographie sur colonne de silice, sous courant d'azote, à moyenne pression (0,5-1,5 bar) avec de l'acétate d'éthyle comme éluant et recristallisation une première fois dans 40 cm3 d'acétonitrile puis dans 35 cm3 de méthyléthylcétone, on obtient 0,8 g de {[(( fluoro-5 indolyl-3) méthyl)-4 pipéridino]-2 éthyl}-2 méthoxy-3 isoindolinone-(RS) fondant à 175° C.

La (bromo-2 éthyl)-2 méthoxy-3 isoindolinone-(RS) peut tre préparée de la manière suivante : à une solution agitée de 2,8 g d'hydroxy-3 (bromo-2 éthyl)-2 isoindolinone-(RS) dans 100 cm3 de méthanol, on ajoute, à une température proche de 0° C, 19,8 cm3 d'acide sulfurique 36N. La solution est chauffée à

ébullition durant 5 heures puis refroidie à une température proche de 20° C. Le précipité formé est filtré et lavé par 30 cm3 de méthanol. Le filtrat est repris par 20 cm3 d'eau distillée et 20 cm3 d'ammoniaque à 20%. La phase organique est extraite par 4 fois 40 cm3 de dichlorométhane, séchée sur sulfate de magnésium anhydre, traitée au noir végétal, filtrée et concentrée à sec, à 40° C sous pression réduite (20mm de mercure - 2,7kPa). L'huile résiduelle est purifiée par flash-chromatographie sur colonne de silice, sous courant d'argon à moyenne pression (0,1-1,5 bar) avec du dichlorométhane comme éluant. On obtient 1,5 g de (bromo-2 éthyl)-2 méthoxy-3 isoindolinone-(RS) sous forme d'une huile jaune utilisée à l'état brut dans les synthèses ultérieures.

EXEMPLE 34

On opère comme dans l'exemple 25 à partir de 2,3 g de N-(chloro-2 éthyl) N-méthyl naphtalène sulfonamide-2, de 1,4 g de [(fluoro-5 indolyl-3) méthyl]-4 pipéridine et de 1,5 g de bicarbonate de sodium dans un mélange de 60 cm3 de diméthylformamide et de 40 cm3 de tétrahydrofuranne. Le mélange est chauffé 36 heures à ébullition puis refroidi à une température voisine de 20° C . Après purification par flash-chromatographie sur colonne de silice, sous courant d'azote, à moyenne pression (0,5-1,5 bar) avec de l'acétate d'éthyle comme éluant et recristallisation dans 90 cm3 d'acétonitrile bouillant , on obtient 1 g de N-{[(( fluoro-5 indolyl-3) méthyl)-4 pipéridino]-2 éthyl} N-méthyl naphtalène sulfonamide-2 fondant à 146° C.

Le N-(chloro-2 éthyl) N-méthyl naphtalène sulfonamide-2 peut être préparé de la manière suivante: on opère comme dans l'exemple 1 pour la préparation du (chloro-2 éthyl)-2 naphto[1,8-cd] isothiazole dioxyde-1,1, à partir de 0,77 g d'hydrure de sodium à 50% en dispersion dans l'huile de vaseline, de 2,25 cm3 de bromo-1 chloro-2 éthane et de 5 g de N-méthyl naphtalène sulfonamide-2 dans 100 cm3 de diméthylformamide. Le milieu réactionnel est agité 5 heures à ébullition, sous courant d'argon, puis refroidi à une température proche de 20° C. Après purification par flash-chromatographie sur colonne de silice, sous courant d'argon, à moyenne pression (0,5-1,5bar) avec un mélange cyclohexane-acétate d'éthyle (80-20 en volumes) comme éluant, on obtient 2,3 g de N-(chloro-2 éthyl) N-méthyl naphtalène sulfonamide-2 fondant à 101° C.

Le N-méthyl naphtalène sulfonamide-2 peut être préparé de la manière suivante: dans un ballon, on condense à -70° C 10 g de méthylamine gaz puis additionne 50 cm3 de tétrahydrofuranne. On ajoute ensuite, à -70° C, une solution de 14,5 g de chlorure de naphtalène sulfonyl-2 dans 50 cm3 de tétrahydrofuranne. L'agitation est maintenue pendant 2 heures et 30 minutes en laissant la température revenir à une température proche de 20° C. Le milieu réactionnel est repris par 200 cm3 d'acétate d'éthyle et 300 cm3 d'eau distillée. La phase organique est décantée, séchée sur du sulfate de magnésium anhydre, filtrée et concentrée à sec à 40° C, sous pression réduite (20mm de mercure; 2,7kPa). On obtient 12,1g de N-méthyl naphtalène sulfonamide-2 fondant à 137° C.

EXEMPLE 35

A une solution de 4g d'isopropényl-3 {[((fluoro-5 indolyl-3) méthyl)-4 pipéridino]-2 éthyl}-1 2H-benzimidazolone-2 dans 50 cm3 d'éthanol, on coule 9,3 cm3 d'acide chlorhydrique 6N à une température voisine de 20° C. La solution est chauffée 1 heure à ébullition puis refroidie à une température proche de 20° C. Le milieu réactionnel est repris par 100 cm3 de dichlorométhane, 30 cm3 d'eau distillée et alcalinisé à pH 9 par de la soude 5N. La phase organique est extraite par 3 fois 30 cm3 de dichlorométhane, lavée par 90 cm3 d'eau distillée, séchée sur sulfate de magnésium anhydre, traitée au noir végétal, filtrée et concentrée à sec à 40° C, sous pression réduite (20mm de mercure, 2,7kPa). Le résidu est recristallisé dans 15 cm3 de méthyléthylcétone. On obtient 0,4 g de {[((fluoro-5 indolyl-3) méthyl)-4 pipéridino]-2 éthyl}-1 2H-benzimidazolinone-2 fondant à 220° C.

L'isopropényl-3 {[((fluoro-5 indolyl-3) méthyl)-4 pipéridino]-2 éthyl}-1 2H-benzimidazolinone-2 peut être préparée de la manière suivante: on opère comme dans l'exemple 25 à partir de 2,4 g d'isopropényl-3 (chloro-2 éthyl)-1 2H-benzimidazolinone-2, de 2,3 g de [(fluoro-5 indolyl-3) méthyl)-4 pipéridine et de 0,8 g de bicarbonate de sodium dans un mélange de 40 cm3 de diméthylformamide et de 20 cm3 de tétrahydrofuranne. Le mélange est chauffé 10 heures à ébullition puis refroidi à une température voisine de 20° C Après purification par flash-chromathographie sur colonne de silice, sous courant d'azote, à moyenne pression (0,5-1,5 bar) avec du dichlorométhane puis de l'acétate d'éthyle comme éluants, on obtient 3 g d'isopropényl-3 {[(( fluoro-5 indolyl-3) méthyl)-4 pipéridino]-2 éthyl}-1 2H-benzimidazolinone-2 fondant à 132° C.

La (chloro-2 éthyl)-1 isopropényl-3 2H-benzimidazolinone-2 peut être préparée de la manière suivante : on opère comme dans l'exemple 1 pour la préparation du (chloro-2 éthyl)-2 naphto[1,8-cd] isothiazole

dioxyde-1,1 , à partir de 6,5 g d'hydrure de sodium à 50% en dispersion dans l'huile de vaseline, de 13,8 cm3 de bromo-1 chloro-2 éthane et de 25 g d'isopropényl-1 2H-benzimidazolinone-2 dans 250 cm3 de diméthylformamide. Le mélange est agité 48 heures à une température proche de 20 °C , sous courant d'argon. Après purification par flash-chromatographie sur colonne de silice, sous courant d'argon à moyenne pression (0,5-1,5bar) avec du dichlorométhane puis un mélange dichlorométhane-acétate d'éthyle (90-10 en volumes) comme éluants, on obtient 20,3 g de (chloro-2 éthyl)-1 isopropényl-3 2H-benzimidazolinone-2 sous forme d'une huile utilisée à l'état brut dans les synthèses ultérieures.

L'isopropényl-1 2H-benzimidazolinone-2 peut être préparée selon la méthode décrite par O.METH-COHN et coll., J.Chem.Soc.,PER-KIN TRANS 1, 261 (1982).

EXEMPLE 36

On opère comme dans l'exemple 25 à partir de 2,7 g de (chloro-2 éthyl)-1 méthyl-3 2H-benzimidazolinone-2, de 3 g de [(fluoro-5 indolyl-3) méthyl)-4 pipéridine et de 1,1 g de bicarbonate de sodium dans un mélange de 35 cm3 de diméthylformamide et de 20 cm3 de tétrahydrofuranne. Le mélange est chauffé 14 heures à ébullition puis refroidi à une température voisine de 20 °C . Après purification par flash-chromathographie sur colonne de silice, sous courant d'azote, à moyenne pression (0,5-1,5 bar) avec du dichlorométhane puis un mélange acétate éthyle-méthanol (97-3 en volumes) comme éluants et recristallisation dans 50 cm3 d'acétonitrile bouillant, on obtient 1,9 g de méthyl-3 {[(( fluoro-5 indolyl-3) méthyl)-4 pipéridino]-2 éthyl}-1 2H-benzimidazolinone-2 fondant à 157 °C.

La (chloro-2 éthyl)-1 méthyl-3 2H-benzimidazolinone-2 peut être préparée selon le brevet US 4254127.

Les médicaments selon l'invention-sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement de la dépression, des troubles obsessionnels, de l'obésité, des troubles de la conduite alimentaire notamment les excès d'alcool, des troubles de l'apprentissage et de la mémoire, des attaques de panique et de la douleur.

Les doses dépendent de l'effet recherché, de la durée du traitement et de la voie d'administration utilisée ; elles sont généralement comprises entre 50 et 300 mg par jour par voie orale pour un adulte avec des doses unitaires allant de 25 à 100 mg de substance active.

D'une façon générale, le médecin déterminera la posologie appropriée en fonction de l'âge, du poids et de tous les autres facteurs propres au sujet à traiter. Les exemples suivants illustrent des compositions selon l'invention :

Exemple A

On prépare, selon la technique habituelle, des gélules dosées à 50 mg de produit actifayant la composition suivante :

| | |
|---|---|
| - {[((fluoro-5 indolyl-3) méthyl)-4 pipéridino]-2 éthyl}-1 benzo[cd]indolone-2 ..... | 50 mg |
| - cellulose ..... | 18 mg |
| - lactose ..... | 55 mg |
| - silice colloïdale ..... | 1 mg |
| - carboxyméthylamidon sodique ..... | 10 mg |
| - talc ..... | 10 mg |
| - stéarate de magnésium ..... | 1 mg |

Exemple B

On prépare, selon la technique habituelle, des comprimés dosés à 50 mg de produit actifayant la composition suivante :

| | |
|---|---|
| - {[((fluoro-5 indolyl-3) méthyl)-4 pipéridino]-2 éthyl}-1 dihydro-5,6 1H,4H-1,2,5-thiadiazolo [4,3,2-ij] quinoléine dioxyde-2,2 ..... | 50 mg |
| - lactose ..... | 104 mg |
| - cellulose ..... | 40 mg |
| - polyvidone ..... | 10 mg |
| - carboxyméthylamidon sodique ..... | 22 mg |
| - talc ..... | 10 mg |
| - stéarate de magnésium ..... | 2 mg |
| - silice colloïdale ..... | 2 mg |
| | - |
| mélange d'hydroxyméthylcellulose, glycérine, oxyde de titane (72-3,5-24,5) q.s.p. 1 comprimé pelliculé terminé à 245 mg | |

Exemple C

On prépare une solution injectable contenant 10 mg de produit actifayant la composition suivante :

| | |
|---|---|
| - {[(indényl-1)méthyl)-4 pipéridino]-3 propyl}-2 naphto[1,8-cd] isothiazole dioxyde-1,1 ..... | 10 mg |
| - acide benzoïque ..... | 80 mg |
| - alcool benzylique ..... | 0,06 cm3 |
| - benzoate de sodium ..... | 80 mg |
| - éthanol à 95 % ..... | 0,4 cm3 |
| - hydroxyde de sodium ..... | 24 mg |
| - propylène glycol ..... | 1,6 cm3 |
| - eau .....q.s.p. | 4 cm3 |

**Revendications**

1 - Composés de formule :

$R_1$ - $(CH_2)_n$ - Het (I)

dans laquelle $R_1$ représente un reste de formule :

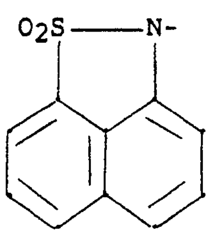
(A)

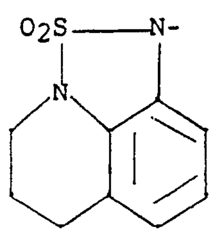
(B)

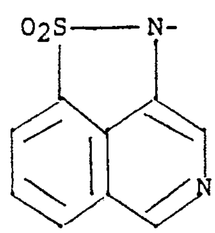
(C)

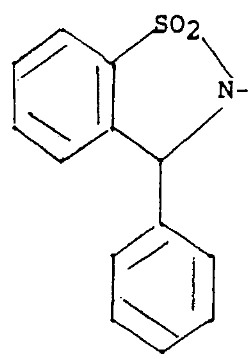
(D)

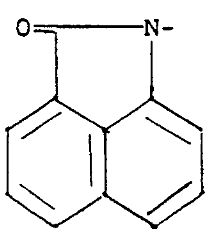
(E)

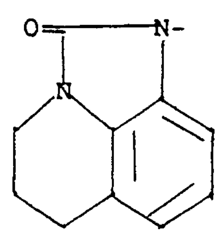
(F)

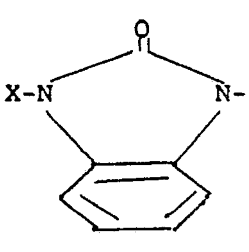
(G)

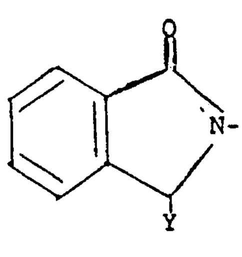
(H)

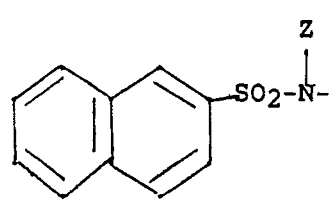
(J)

- X représente un atome d'hydrogène ou un radical alkyle,
- Y représente un atome d'hydrogène ou un radical alkyle ou alcoxy,
- Z représente un radical alkyle
- n est égal à 2 ou 3,
- Het représente un radical pipéridino substitué en (position -4 par un radical indénylidène-1, indényl-1, indanyl-1 ou par une chaîne $-(CH_2)_m-R_2$ ou $=CH-R_2$), tétrahydro-1,2,3,6 pyridyl-1 substitué (en position -4 par une chaîne $-(CH_2)_m-R_2$) ou pipérazinyl-1 substitué (en position -4 par une chaîne $-(CN_2)_m-R_2$),
- m est égal à 1 ou 2,
- $R_2$ représente un radical indolyl-2 ou -3 (éventuellement substitué par un atome d'halogène et/ou sur l'atome d'azote par un radical alkyle), indanyl-1 ou -2, indényl-1 ou -2, quinolyl-3, chromanyl-3, tétrahydro-1,2,3,4 5H pyrido [4,3-b] indolyl-2 (éventuellement substitué par un atome d'halogène), (tétrahydro-1,2,3,4 quinolyl)-3, (tétrahydro-1,2,3,4 naphtyl)-2 (éventuellement substitué par un atome d'halogène) ou indolyl-1,ainsi que leurs sels avec des acides minéraux ou organiques et leurs racémiques et leurs énantiomères lorsqu'ils contiennent au moins un centre asymétrique, étant entendu que les radicaux alkyle et alcoxy contiennent 1 à 4 atomes de carbone en chaîne droite ou ramifiée.

2 - Composés de formule (I) selon la revendication 1 pour lesquels les atomes d'halogène sont des atomes de fluor.

3 - Composés de formule (I) selon les revendications 1 ou 2 pour lesquels Het représente un radical pipéridino substitué en position-4 par une chaîne $-(CH_2)_m-R_2$ et $R_2$ représente un radical (fluoro-5 indolyl)-3

4 - {[((fluoro-5 indolyl-3) méthyl)-4 pipéridino]-2 éthy}-1 dihydro-5,6 1H,4H-1,2,5-thiadiazolo [4,3,2-ij] quinoléine dioxyde-2,2 et ses sels d'addition avec un acide minéral ou organique.

5 - Procédé de préparation des composés de formule (I) selon la revendication à l'exception de ceux pour lesquels $R_1$ représente un reste de formule (G) dans laquelle X représente un atome d'hydrogène, caractérisé en ce que l'on fait réagir un dérivé halogéné de formule :

$R_1-(CH_2)_n-Hal$ (II)

dans laquelle $R_1$ a les mêmes significations que précédemment, n a les mêmes significations que dans la revendication 1 et Hal représente un atome d'halogène, sur un dérivé de formule :

H - Het (III)

dans laquelle Het a les mêmes significations que dans la revendication 1, isole le produit et le transforme éventuellement en sel d'addition avec un acide minéral ou organique.

6 - Procédé de préparation des composés de formule (I) selon la revendication 1 pour lesquels $R_1$ représente un reste de formule (G) dans laquelle X représente un atome d'hydrogène, caractérisé en ce que l'on hydrolyse un dérivé de formule :

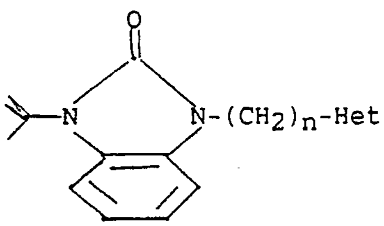

(VI)

dans laquelle n et Het ont les mêmes significations que dans la revendication 1, isole le produit et le transforme éventuellement en sel d'addition avec un acide minéral ou organique.

7 - Procédé de préparation des composés de formule (I) selon la revendication 1 pour lesquels $R_2$ représente un radical indolyl-2 ou -3 substitué sur l'atome d'azote par un radical alkyle et éventuellement substitué par un atome d'halogène caractérisé en ce que l'on alkyle un composé de formule (I) correspondant pour lequel $R_2$ représente un radical indolyl-2 ou -3 éventuellement substitué par un atome d'halogène, isole le produit et le transforme éventuellement en sel d'addition avec un acide minéral ou organique.

8 - Procédé de préparation des composés de formule (I) selon la revendication 1 pour lesquels $R_1$ représente un reste de formule (H) dans laquelle Y représente un atome d'hydrogène, caractérisé en ce que l'on réduit un dérivé de formule :

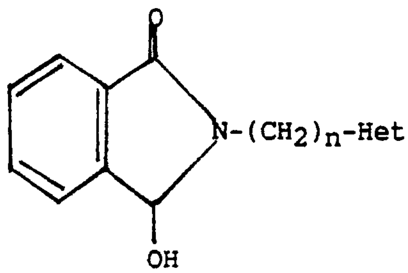

(VII)

dans laquelle n et Het ont les mêmes significations que dans la revendication 1, isole le produit et le transforme éventuellement en sel d'addition avec un acide minéral ou organique.

9 - Procédé de préparation des composés de formule (I) pour lesquels Het représente un radical pipéridino substitué en position -4 par un radical indènylidène-1 caractérisé en ce que l'on fait réagir un dérivé de formule :

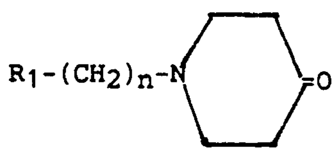

(VIII)

dans laquelle n et $R_1$ ont les mêmes significations que dans la revendication 1 avec un dérivé métallique de l'indène, isole le produit et le transforme éventuellement en sel avec un acide minéral ou organique.

10 - Médicaments caractérisés en ce qu'ils contiennent comme matière active au moins un composé de formule (I) selon l'une des revendications 1, 2, 3 ou 4.

11 - Médicaments selon la revendication 10 pour le traitement et la prévention des désordres liés à la CCK.

Revendications pour les Etats contractants suivants: ES, GR

1 - Procédé de préparation des composés de formule :

$R_1$-$(CH_2)_n$-Het (I)

dans laquelle $R_1$ représente un reste de formule :

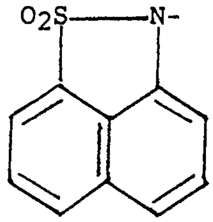

(A)

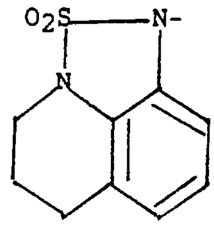

(B)

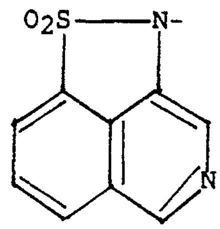

(C)

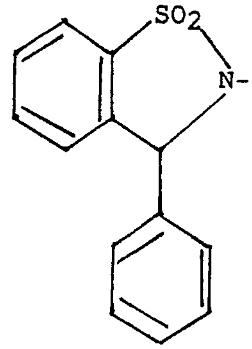

(D)

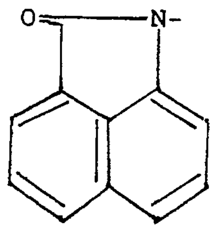

(E)

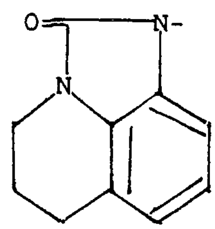

(F)

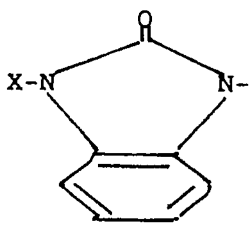

(G)

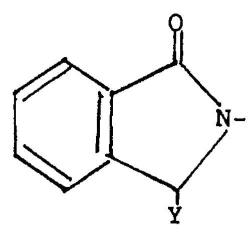

(H)

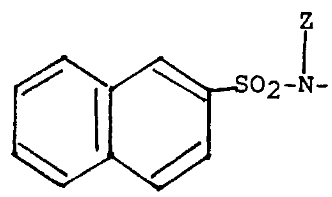

(J)

- X représente un atome d'hydrogène ou un radical alkyle,
- Y représente un atome d'hydrogène ou un radical alkyle ou alcoxy
- Z représente un radical alkyle,
- n est égal à 2 ou 3,
- Het représente un radical pipéridino substitué en (position -4 par un radical indénylidène-1, indényl-1, indanyl-1 ou par une chaîne $-(CH_2)_m-R_2$ ou $=CH-R_2$), tétrahydro-1,2,3,6 pyridyl-1 substitué (en position -4 par une chaîne $-(CH_2)_m-R_2$) ou pipérazinyl-1 substitué (en position -4 par une chaîne $-(CH_2)_m-R_2$),
- m est égal à 1 ou 2,
- $R_2$ représente un radical indolyl-2 ou -3 (éventuellement substitué par un atome d'halogène et/ou sur l'atome d'azote par un radical alkyle), indanyl-1 ou -2, indényl-1 ou -2, quinolyl-3, chromanyl-3, tétrahydro-1,2,3,4 5H pyrido [4,3-b] indolyl-2 (éventuellement substitué par un atome d'halogène), (tétrahydro-1,2,3,4 quinolyl)-3, (tétrahydro-1,2,3,4 naphtyl)-2 (éventuellement substitué (par un atome d'halogène) ou indolyl-1,ainsi que leurs sels avec des acides minéraux ou organiques et leurs racémiques et leurs énantiomères lorsqu'ils contiennent au moins un centre asymétrique, étant entendu que les radicaux alkyle et alcoxy contiennent 1 à 4 atomes de carbone en chaîne droite ou ramifiée, caractérisé en ce que :

A - Pour la préparation des composés de formule (I) à l'exception de ceux pour lesquels $R_1$ représente un reste de formule (G) dans laquelle X représente un atome d'hydrogène, on fait réagir un dérivé halogéné de formule :

$R_1-(CH_2)_n-Hal$ (II)

dans laquelle $R_1$ a les mêmes significations que précédemment, n a les mêmes significations que ci-dessus et Hal représente un atome d'halogène, sur un dérivé de formule :

H-Het (III)

dans laquelle Het a les mêmes significations que ci-dessus puis isole le produit et le transforme éventuellement en sel d'addition avec un acide minéral ou organique,

B - Pour la préparation des composés de formule (I) pour lesquels $R_1$ représente un reste de formule (G) dans laquelle X représente un atome d'hydrogène, on hydrolyse un dérivé de formule :

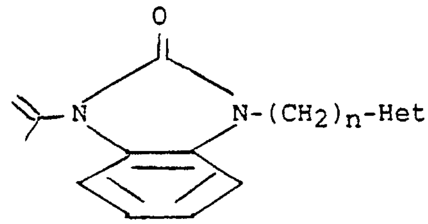

(VI)

dans laquelle n et Het ont les mêmes significations que ci-dessus, isole le produit et le transforme éventuellement en sel d'addition avec un acide minéral ou organique.

C - Pour la préparation des composés de formule (I) pour lesquels $R_2$ représente un radical indolyl-2 ou -3 substitué sur l'atome d'azote par un radical alkyle et éventuellement substitué par un atome d'halogène, on alkyle un composé de formule (I) correspondant pour lequel $R_2$ représente un radical indolyl-2 ou -3 éventuellement sunstitué par un atome d'halogène, isole le produit et le transforme éventuellement en sel d'addition avec un acide minéral ou organique.

D - Pour la préparation des composés de formule (I) pour lesquels $R_1$ représente un reste de formule (H) dans laquelle Y représente un atome d'hydrogène, on réduit un dérivé de formule :

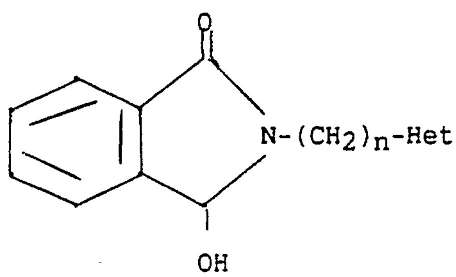

(VII)

dans laquelle n et Het ont les mêmes significations que ci-dessus, isole le produit et le transforme éventuellement en sel d'addition avec un acide minéral ou organique.

E - Pour la préparation des composés de formule (I) pour lesquels Het représente un radical pipéridino substitué en position-4 par un radical indénylidène-1, on fait réagir un dérivé de formule :

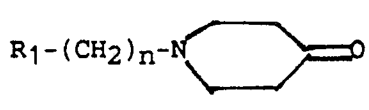

(VIII)

dans laquelle n et $R_1$ ont les mêmes significations que ci-dessus avec un dérivé métallique de l'indène, isole le produit et le transforme éventuellement en sel avec un acide minéral ou organique.

2 - Procédé selon la revendication 1 pour la préparation des composés de formule (I) pour lesquels les atomes d'halogène sont des atomes de fluor.

3 - Procédé selon la revendication 1 pour la préparation des composés de formule (I) pour lesquels Het représente un radical pipéridino substitué en position-4 par une chaîne $-(CH_2)_m-R_2$ et $R_2$ représente un radical (fluoro-5 indolyl)-3.

4 - Prccédé selon la revendication 1 pour la préparation du {[((fluoro-5 indolyl-3) méthyl)-4 pipéridino]-2

éthyl}-1 dihydro-5,6 1H, 4H-1,2,5-thiadiazolo [4,3,2-ij] quinoléine dioxyde-2,2.